# EUROPEAN PATENT APPLICATION

(11) **EP 4 470 466 A1**
(43) Date of publication of application: **04.12.2024**
(21) Application number: 23746783.2
(22) Date of filing: 18.01.2023
(51) Int. Cl.: A61B 5/256, A61B 5/265, A61B 5/274

(54) **FLEXIBLE SHEET ELECTRODE, WEARABLE BIOELECTRODE, AND BIOSENSOR**

(30) Priority: 26.01.2022 JP 2022010456
(71) Applicant: SUMITOMO BAKELITE CO., LTD., Tokyo 140-0002 (JP)
(72) Inventor: OKADA, Jun, Tokyo 140-0002 (JP); YAMANOI, Yumiko, Tokyo 140-0002 (JP); SATO, Motoki, Tokyo 140-0002 (JP)
(74) Representative: Ter Meer Steinmeister & Partner
(86) International application number: PCT/JP2023/001321
(87) International publication number: WO 2023/145569

(57) **Abstract**

Provided is a flexible sheet electrode including a flexible base material, and an elastomer electrode layer provided over the flexible base material, in which the elastomer electrode layer is formed over an outside of the flexible base material and includes a lamination layer including a conductive elastomer layer, and in a case where a tensile test is performed on the flexible sheet electrode according to a predetermined procedure, a 100% tensile test force is greater than or equal to 5.0 N and less than or equal to 10.0 N.

## Description

### TECHNICAL FIELD

The present invention relates to a flexible sheet electrode, a wearable bioelectrode, and a biosensor.

### BACKGROUND ART

Various developments have been made for sheet electrodes so far. As this kind of technology, for example, a technology disclosed in Patent Document 1 is known. Patent Document 1 discloses an electrode member formed such that a conductive polymer is carried on a surface of a single fiber and/or in a gap between single fibers constituting a fiber structure (Claim 1 and the like of Patent Document 1). The examples of the document describe, as a method of allowing the conductive polymer to be carried by fiber structure, a method of gravure-coating the fiber structure with a dispersion liquid obtained by dispersing a conductive polymer such as PEDOT/PSS and a binder in a solvent such that the coating amount reaches about 15 g/m² (about 1.5 mg/cm²).

### RELATED DOCUMENT

### PATENT DOCUMENT

[Patent Document 1] International Publication No. WO2015-115440

### SUMMARY OF THE INVENTION

### TECHNICAL PROBLEM

However, the configuration in which a conductive elastomer layer is used as a sheet-like electrode part has not been sufficiently examined in the electrode member of Patent Document 1.

As a result of examination conducted by the present inventors, it has been found that a sheet electrode including a conductive elastomer layer has room for improvement in terms of conduction characteristics and ease of deformation after washing.

### SOLUTION TO PROBLEM

As a result of further examination, the present inventors have found that, in a flexible sheet electrode including a flexible base material and a lamination layer formed over an outside of the flexible base material and including a conductive elastomer layer, conduction characteristics of the flexible sheet electrode after washing and ease of follow-up deformation of the flexible sheet electrode can be stably evaluated by using a tensile test force in a tensile test as an index.

As a result of intensive examination based on such findings, it has been found that an increase in surface resistivity of the flexible sheet electrode after repeated washing can be suppressed and the conduction characteristics after washing can be improved by using the tensile test force during elongation in a case of elongating the flexible sheet electrode as an index and setting such an index to be greater than or equal to a predetermined value, and ease of follow-up deformation of the flexible sheet electrode can be improved by setting the index to be less than or equal to a predetermined value, thereby completing the present invention.

According to one aspect of the present invention, there are provided a flexible sheet electrode, a wearable bioelectrode, and a biosensor.
1. A flexible sheet electrode including: a flexible base material; and an elastomer electrode layer provided over the flexible base material, in which the elastomer electrode layer is formed over an outside of the flexible base material and includes a lamination layer including a conductive elastomer layer, and in a case where a tensile test is performed on the flexible sheet electrode according to the following procedure, a 100% tensile test force is greater than or equal to 5.0 N and less than or equal to 10.0 N.

### (Procedure)

A sample test piece having a rectangular shape is prepared from the flexible sheet electrode.

In a case where the elastomer electrode layer of the sample test piece is held by chucks of a tensile testing machine such that a center of the elastomer electrode layer is positioned at a center between the chucks, and a distance between the chucks is elongated from 0% to 250% under a condition of a tensile rate of 250 mm/min, a tension (N) is measured during elongation of the distance by X%, and a value of the tension is defined as the X% tensile test force.

2. The flexible sheet electrode according to 1., in which in a case where the tensile test is performed on the flexible sheet electrode according to the procedure, a 20% tensile test force is greater than or equal to 1.5 N and less than or equal to 5.5 N.

3. The flexible sheet electrode according to 1. or 2., further including: an impregnation layer formed over an inside of the flexible base material, in which the impregnation layer includes the conductive elastomer layer and/or an insulating elastomer layer.

4. The flexible sheet electrode according to any one of 1. to 3., in which the conductive elastomer layer contains a conductive filler and a non-conductive filler.

5. The flexible sheet electrode according to 4., in which the conductive filler contains scale-like silver powder.

6. The flexible sheet electrode according to 4. or 5., in which the non-conductive filler contains silica particles.

7. The flexible sheet electrode according to any one of 4. to 6., in which a content of the conductive filler in the conductive elastomer layer is greater than or equal to 50% by mass and less than or equal to 90% by mass.

8. The flexible sheet electrode according to any one of 1. To 7., in which the conductive elastomer layer contains silicone rubber.

9. The flexible sheet electrode according to any one of 1. to 8., in which the flexible base material is woven fabric or knitted fabric.

10. A wearable bioelectrode including: the flexible sheet electrode according to any one of 1. to 9.

11. A biosensor comprising: the wearable bioelectrode according to 10.

### ADVANTAGEOUS EFFECTS OF INVENTION

According to the present invention, it is possible to provide a flexible sheet electrode with excellent conduction characteristics and excellent ease of deformation after washing, and a wearable bioelectrode and a biosensor, which use the flexible sheet electrode.

### BRIEF DESCRIPTION OF THE DRAWINGS

Figs. 1A and 1B are views schematically showing an example of a configuration of a flexible sheet electrode according to the present embodiment (Fig. 1A is a cross-sectional view and Fig. 1B is a top view).
Figs. 2A and 2B are cross-sectional views schematically showing an example of a modification example of the flexible sheet electrode.
Fig. 3 is a top view schematically showing an example of a configuration of a wearable bioelectrode according to the present embodiment.
Fig. 4 is a cross-sectional view taken along the line B-B of Fig. 3.
Fig. 5 is a schematic view for describing an example of a configuration of a biosensor according to the present embodiment.
Fig. 6 is a schematic view for describing a tensile test.

### DESCRIPTION OF EMBODIMENTS

Hereinafter, embodiments of the present invention will be described with reference to the accompanying drawings. Further, in all the drawings, the same constituent elements are denoted by the same reference numerals, and the description thereof will not be repeated. In addition, the drawings are schematic views, and the dimensional ratios thereof do not match the actual dimensional ratios.

The outline of the flexible sheet electrode according to the present embodiment will be described.

A flexible sheet electrode according to the present embodiment includes a flexible base material, and an elastomer electrode layer provided over the flexible base material, in which the elastomer electrode layer is formed on the outside of the flexible base material and includes a lamination layer including a conductive elastomer layer, and in a case where a tensile test is performed on the flexible sheet electrode, a 100% tensile test force is greater than or equal to 5.0 N and less than or equal to 10.0 N.

According to the present embodiment, a washable flexible sheet electrode can be realized.

The tensile test is performed on the flexible sheet electrode according to the following procedure, and the stress at 20% elongation and the test force (N) at 100% elongation are respectively measured as a 20% tensile test force and a 100% tensile test force.

### (Measurement procedure of tensile test)

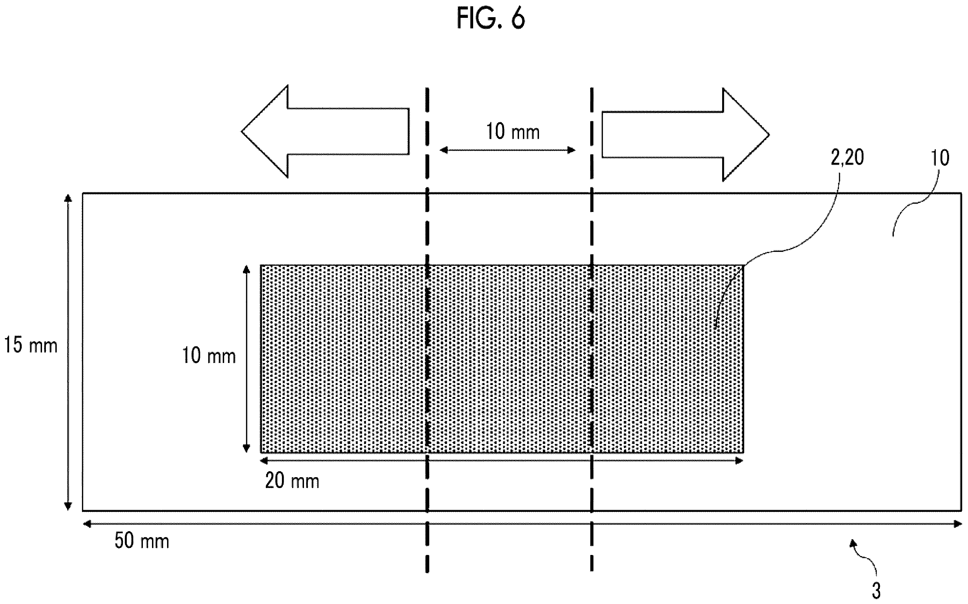

Fig. 6 is a schematic view for describing the tensile test.

First, a sample test piece having a rectangular shape is prepared from the flexible sheet electrode.

Subsequently, the elastomer electrode layer of the sample test piece is held by chucks of a tensile testing machine such that the center of the elastomer electrode layer is positioned at the center between the chucks.

Thereafter, in a case where the distance between the chucks is elongated from 0% to 250% under a condition of a tensile rate of 250 mm/min, the tension (N) is measured during elongation of the distance by X%, and the value thereof is defined as the X% tensile test force.

As an example of the dimensions of the sample test piece having a rectangular shape, a printed portion (the conductive elastomer layer on the surface side of the elastomer electrode layer) has a size of 10 mm × 20 mm, and the flexible base material has a size of 15 mm × 50 mm.

In addition, in a case where the distance between the chucks is set to 10 mm and the distance between the chucks is elongated from the initial 10 mm to 35 mm, the distance between the chucks is elongated by 250%.

Further, the tensile test force may be a tension measured after each elongated state is held for 1 minute.

The upper limit of the 100% tensile test force is less than or equal to 10.0 N, preferably less than or equal to 9.5, and more preferably less than or equal to 9.0. In this manner, ease of followability of the flexible sheet electrode can be improved.

The lower limit of the 100% tensile test force is 5.0 N or greater, preferably 5.5 or greater, and more preferably 6.0 or greater. In this manner, the conduction characteristics after washing can be improved.

The upper limit of the 20% tensile test force is, for example, less than or equal to 5.5 N, preferably less than or equal to 5.3 N, and more preferably less than or equal to 5.0 N. In this manner, the washing durability can be improved.

The lower limit of the 20% tensile test force is, for example, 1.5 N or greater, preferably 1.8 or greater, and more preferably 2.0 or greater. In this manner, ease of the initial followability of the flexible sheet electrode can be maintained.

The flexible sheet electrode can be used for various applications, and one of the applications is a wearable bioelectrode. The wearable bioelectrode according to the present embodiment includes the above-described flexible sheet electrode.

The wearable bioelectrode can detect potential fluctuations from a living body, such as a heartbeat, muscle activity, or nervous system activity. For example, the wearable bioelectrode may be configured to be used for measuring at least one biological potential from among a cardiac potential, a muscle potential, and a skin potential.

According to the present embodiment, a wearable bioelectrode that can be elongated and/or bent can be realized.

In such a wearable bioelectrode, even in a case of deformation such as stretching and contraction or bending of the base material, breakage in the flexible sheet electrode is suppressed, and the biological potential can be stably measured. Further, even in a case of the deformation of the base material, occurrence of peeling of the flexible sheet electrode from the flexible base material is suppressed, and the adhesiveness therebetween is maintained.

The wearable bioelectrode can be used as a wearable device that can be mounted on any one of the body or the clothing. Such a wearable bioelectrode can follow the surface shape of the body or the movement of the body due to the elasticity thereof. In this case, the wearable bioelectrode may be mounted on the body directly or through a body mounting member (clothing). The clothing with the wearable bioelectrode has, for example, a configuration in which the wearable bioelectrode is sewn to the clothing or a configuration in which the wearable bioelectrode is used in a part of the clothing.

The wearable bioelectrode can further include a connector, an electronic component, or the like, and can constitute a biosensor that can be connected to an external device. The biosensor is wearable. A biological signal measurement system according to various applications can be constructed by analyzing the biological potential such as a cardiac potential detected from the biosensor.

Such a wearable bioelectrode, the biosensor using the wearable bioelectrode, and the biological signal measurement system are expected to be used in various fields such as physical diagnosis, health management, fitness, rehabilitation, and nursing care.

Hereinafter, the configurations of the flexible sheet electrode and the wearable bioelectrode according to the present embodiment will be described in detail.

### <Flexible sheet electrode>

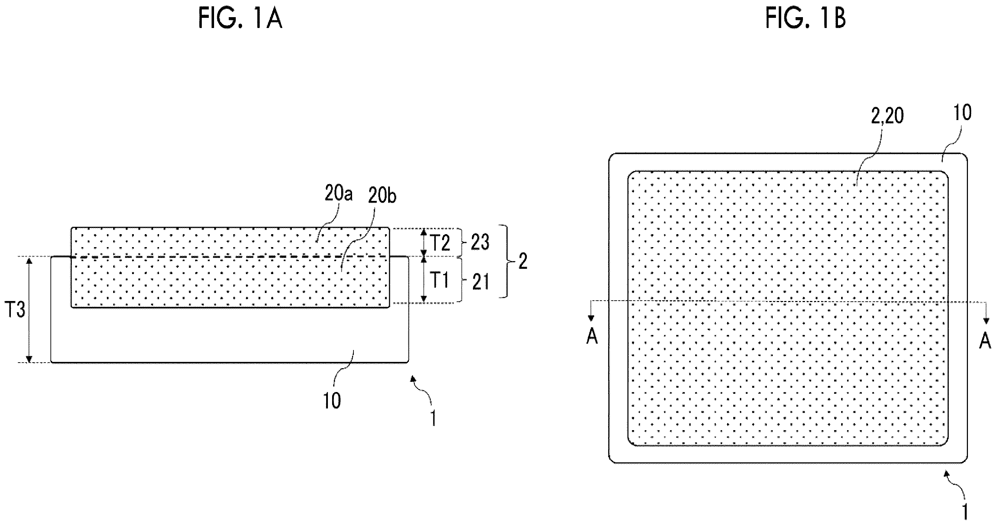

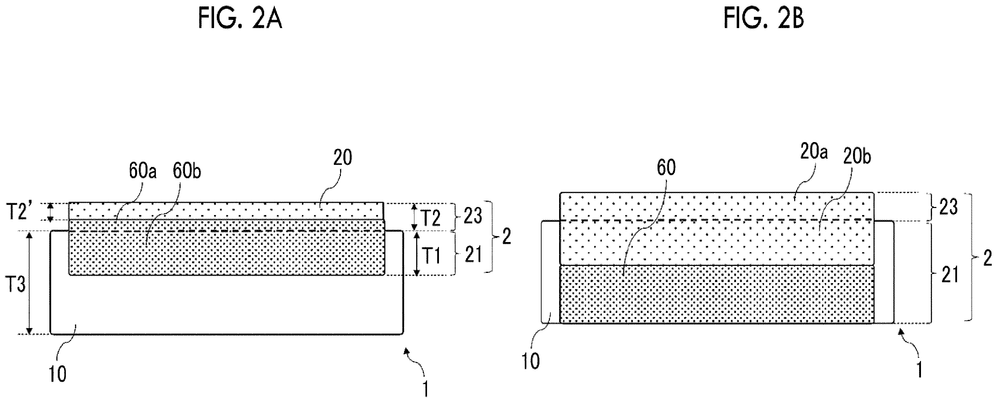

Figs. 1A and 1B are cross-sectional view and top view showing an example of a configuration of a flexible sheet electrode 1. Fig. 1A is a cross-sectional view of the flexible sheet electrode 1 taken along the line A-A of Fig. 1B, and Fig. 1B is a top view of the flexible sheet electrode 1. Fig. 2A and Fig. 2B are cross-sectional views showing a modification example of the flexible sheet electrode 1.

The flexible sheet electrode 1 includes a flexible base material 10 and an elastomer electrode layer 2 provided over the flexible base material 10.

The elastomer electrode layer 2 includes at least a lamination layer 23 formed such that the inside of the flexible base material 10 is not impregnated with the lamination layer 23 and preferably an impregnation layer 21 formed such that the inside of the flexible base material 10 is impregnated with the lamination layer 23 and the impregnation layer 21.

From the viewpoint of a cross-sectional view of the flexible sheet electrode 1 in a thickness direction, the lamination layer 23 is a layer formed on the outside of the flexible base material 10, and the impregnation layer 21 is a layer formed on the inside of the flexible base material 10.

The elastomer electrode layer 2 is not particularly limited as long as the elastomer electrode layer 2 includes the lamination layer 23 including a conductive elastomer layer 20, and according to one preferable aspect, the elastomer electrode layer 2 includes the lamination layer 23 including the conductive elastomer layer 20 on at least a part of the surface thereof.

The lamination layer 23 covers a part or the entire surface of the flexible base material 10, but may only cover the surface to cover at least a measurement range of the biological potential. From the viewpoint of measuring the biological potential, the surface of the lamination layer 23 may be formed of a smooth surface.

The elastomer electrode layer 2 may include the impregnation layer 21 that is bonded to the lamination layer 23. The impregnation layer 21 may be formed to include the conductive elastomer layer 20 and/or an insulating elastomer layer 60.

The lamination layer 23 of Fig. 1A is formed of only a conductive elastomer layer 20a, but the present invention is not limited thereto, and the lamination layer 23 may include an insulating elastomer layer 60a in a portion in the thickness direction as shown in Fig. 2A. That is, the lamination layer 23 may include the conductive elastomer layer 20 and another insulating elastomer layer 60b on the inner side of the conductive elastomer layer 20. In this manner, the flexibility of the flexible sheet electrode can be improved.

The impregnation layer 21 of Fig. 1A may be formed of only a conductive elastomer layer 20b, but the present invention is not limited thereto, and the impregnation layer 21 may be formed of only the insulating elastomer layer 60b as shown in Fig. 2A or may include the conductive elastomer layer 20b in a part thereof in the thickness direction as shown in Fig. 2B.

In a case where the lamination layer 23 is formed of a multilayer, among the outermost layers of the lamination layer 23, the electrode region in the elastomer electrode layer 2 may be formed of the conductive elastomer layer 20. In other words, the surface region of the lamination layer 23 in a top view is partially or entirely formed of the conductive elastomer layer 20.

In one cross section of the flexible sheet electrode 1 of Fig. 1A, the thickness of the impregnation layer 21 is defined as T1, the thickness of the lamination layer 23 is defined as T2, and the thickness of the flexible base material 10 is defined as T3. In Fig. 2A, the thickness of the lamination layer 23 is defined as T2, and the thickness of the conductive elastomer layer 20 in the lamination layer 23 is defined as T2'.

As a method of measuring the thickness, a method of determining the thickness of each layer such as the thickness (T1) of the impregnation layer 21 in which the inside of the flexible base material 10 is impregnated with the elastomer electrode layer 2, the thickness (T2) of the lamination layer 23 in which the inside of the flexible base material 10 is not impregnated with the elastomer electrode layer 2, or the thickness (T3) of the flexible base material 10 based on an SEM image in a predetermined region with a size of 1 mm × 1 mm, acquired by cutting the flexible sheet electrode 1 in the lamination direction and observing one cross section thereof with an electron microscope can be employed.

The electrode is formed such that the T1 and the T2 satisfy, for example, 0.1 ≤ T1/T2 ≤ 100.

The lower limit of T1/T2 is, for example, 0.1 or greater, preferably 0.2 or greater, and more preferably 0.3 or greater. In this manner, the adhesiveness between the conductive elastomer layer and the flexible base material and the washing resistance are improved.

The upper limit of T1/T2 is, for example, less than or equal to 100, preferably less than or equal to 50, and more preferably less than or equal to 30. In this manner, the conductivity can be increased.

The electrode is formed such that the T2 and the T2' satisfy, for example, 0.02 ≤ T2'/T2 ≤ 1.

The lower limit of T2'/T2 is, for example, 0.02 or greater, preferably 0.05 or greater, and more preferably 0.1 or greater. In this manner, the conductivity and the washing durability can be improved.

The upper limit of T2'/T2 is, for example, less than or equal to 1, preferably less than or equal to 0.8, and more preferably 0.5. In this manner, the flexibility of the flexible sheet electrode is increased.

The electrode is formed such that the T1 and the T3 satisfy, for example, 0.01 ≤ T1/T3 ≤ 1.

The lower limit of T1/T3 is, for example, 0.01 or greater, preferably 0.1 or greater, and more preferably 0.3 or greater. In this manner, the adhesiveness between the conductive elastomer layer and the flexible base material and the washing resistance are improved.

The upper limit of T1/T3 is, for example, less than or equal to 1, preferably less than or equal to 0.8, and more preferably less than or equal to 0.7. In this manner, a decrease in flexibility of the flexible sheet electrode can be suppressed.

The lower limit of the adhesion amount of the conductive elastomer layer 20 in the elastomer electrode layer 2 is, for example, 10 mg/cm² or greater, preferably 15 mg/cm² or greater, and more preferably 20 mg/cm² or greater. In this manner, the conductivity can be increased.

The upper limit of the adhesion amount of the conductive elastomer layer 20 in the elastomer electrode layer 2 is, for example, less than or equal to 100 mg/cm², preferably less than or equal to 80 mg/cm², and more preferably less than or equal to 60 mg/cm². In this manner, a decrease in flexibility of the flexible sheet electrode can be suppressed.

As shown in Fig. 1B, the elastomer electrode layer 2 is formed in a sheet shape, and may be formed, as a top view shape, in a polygonal shape such as a rectangular shape, a circular shape, or an elliptical shape as viewed in an upper surface direction of the flexible sheet electrode 1.

In the present specification, in a case where the thickness of the lamination layer 23 of the elastomer electrode layer 2 is defined as D (mm) and the area of one surface 22 of the lamination layer 23 in the top view is defined as S (mm²), S and D in the sheet shape satisfy, for example, 50 ≤ S/D, preferably 200 ≤ S/D, and more preferably 400 ≤ S/D. The upper limit of S/D may be set according to the measurement site of a subject, and is not particularly limited, and may be, for example, S/D ≤ 10⁷.

The elastomer electrode layer 2 and the conductive elastomer layer 20 have elasticity.

The elasticity in the present specification is represented by an elongation ratio in a case of elongation in a predetermined direction. For example, in the top view of Fig. 1B, a direction in which the conductive elastomer layer 20, specifically, the conductive elastomer layer 20 has a maximum length may be employed as the predetermined direction. In a case where the top view shape of the conductive elastomer layer 20 is a rectangular shape, the conductive elastomer layer 20 may be elongated in a diagonal direction.

The expression "the conductive elastomer layer 20 has elasticity" in a case where the conductive elastomer layer 20 is elongated in the extending direction denotes that the layer can be elongated such that the elongation ratio thereof is, for example, greater than or equal to 10%, preferably greater than or equal to 20%, and more preferably greater than or equal to 50% and indicates a state where the conductive elastomer layer 20 is not broken at the elongation ratio.

Further, the resistivity such as the surface resistivity or the like may be obtained by measuring the surface of the conductive elastomer layer 20 using a four-probe method.

The conductive elastomer layer 20 may be formed by a printing method using a conductive paste that contains a conductive elastomer. That is, according to an example, the conductive elastomer layer 20 is formed of a printing layer of a conductive paste. Therefore, it is possible to provide a flexible sheet electrode with excellent design freedom in the sheet electrode design.

The flexible base material 10 is not particularly limited as long as the base material is a base material that can be elongated and/or bent and deformed, but may be formed of, for example, a fiber base material, a porous elastomer base material, or an elastomer base material. A base material containing an insulating elastomer described below may be used as the elastomer base material.

Examples of the fiber structure material of the fiber base material include natural fibers such as plant fibers and animal fibers; and chemical fibers such as inorganic fibers, regenerated fibers, semisynthetic fibers, and synthetic fibers. These may be used alone or in combination of two or more kinds thereof. Among these, from the viewpoint of the durability, chemical fibers may be used, or synthetic fibers such as acryl, polyester, nylon, and polyurethane may be used.

Known fiber base materials can be used as the fiber base material, and the fiber base material may be formed of, for example, any one or both of the insulating material and the conductive material.

The lower limit of the basis weight of the fiber base material is, for example, 10 g/m² or greater, preferably 20 g/m² or greater, and more preferably 30 g/m² or greater. In this manner, the mechanical strength is increased.

Meanwhile, the upper limit of the basis weight of the fiber base material is, for example, less than or equal to 500 g/m², preferably less than or equal to 400 g/m², and more preferably less than or equal to 350 g/m². In this manner, a decrease in flexibility can be suppressed. Further, the degree of impregnation of the conductive elastomer layer can be increased.

The structure of the fiber base material is not limited, but woven fabric or knitted fabric may be used from the viewpoint of the flexibility.

The upper limit of the thickness T3 of the flexible base material 10 can be set according to the applications thereof, and may be set to, for example, less than or equal to 10 mm and preferably less than or equal to 1 mm. Further, from the viewpoint of the applications of the wearable device, the upper limit thereof is more preferably less than or equal to 600 pm. In a case where the upper limit of the thickness thereof is set to less than or equal to 600 µm, a thin film sheet-like wearable bioelectrode can be realized.

From the viewpoint of the mechanical strength, the lower limit of the thickness T3 of the flexible base material 10 is, for example, 10 µm or greater, preferably 50 um or greater, and more preferably 100 um or greater.

As an example of a method of producing the flexible sheet electrode 1, a method using screen printing will be described.

First, a support is placed on a working stand, and the flexible base material 10 is placed on the support.

Subsequently, a mask having a predetermined opening pattern shape is disposed on the flexible base material 10.

Next, the flexible base material 10 is coated with an insulating paste and/or a conductive paste via a mask using a squeegee. The coating order of the insulating paste and the conductive paste can be appropriately changed according to the laminated structure of the elastomer electrode layer 2. A drying treatment may be performed during the coating as necessary.

Thereafter, the insulating paste and/or the conductive paste is subjected to a curing treatment. In a case where the insulating paste and/or the conductive paste contains the silicone rubber-based curable composition, for example, a curing temperature can be set to be in a range of 160°C to 220°C, and a curing time can be set to be in a range of 1 hour to 3 hours. The mask is removed before or after the curing treatment.

Thereafter, the support is separated from the flexible base material 10 to obtain the flexible sheet electrode 1.

In the present embodiment, the thickness T1 of the impregnation layer 21, the thickness T2 of the lamination layer 23, the thickness of the conductive elastomer layer 20 in the impregnation layer 21, the thickness of the insulating elastomer layer 60 in the lamination layer 23, and the adhesion amount of the conductive elastomer layer 20 and/or the insulating elastomer layer 60 can be controlled, for example, by appropriately selecting a method of preparing the flexible sheet electrode 1 or the like. Among these, for example, appropriate selection of the thickness of the mask, the number of lamination layers of the mask, the number of times of coating, the solid content concentration of the insulating paste or the conductive paste, and the like is considered as a factor for setting the thicknesses and the adhesion amounts described above to be in desired numerical ranges.

### <Wearable bioelectrode>

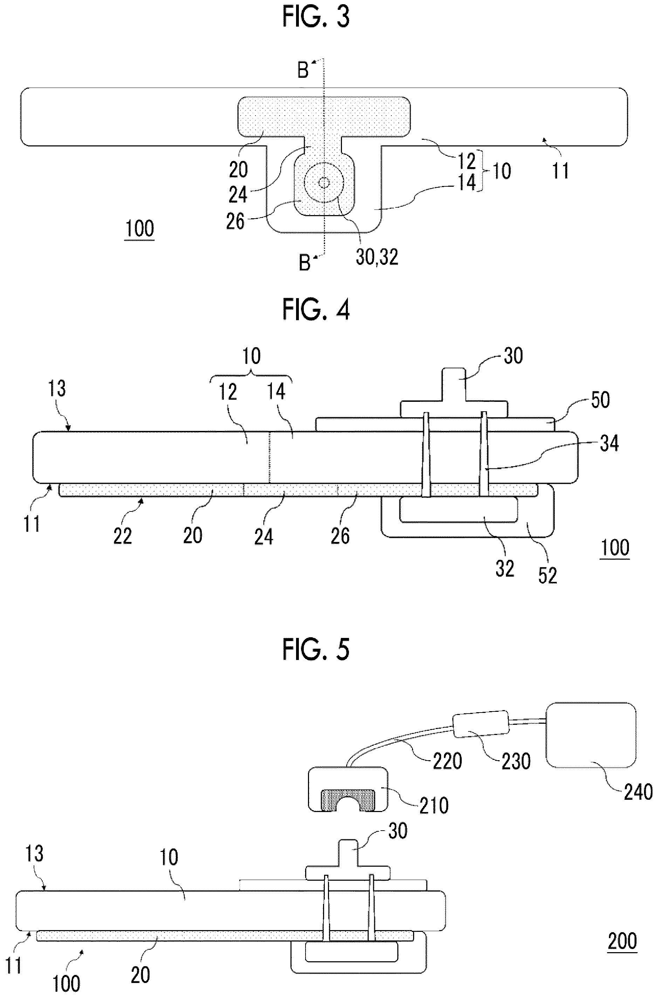

Fig. 3 is a top view showing an example of a configuration of a wearable bioelectrode 100. Fig. 4 is a cross-sectional view taken along the line B-B of Fig. 3.

The wearable bioelectrode 100 includes the flexible sheet electrode 1 including the flexible base material 10 and the elastomer electrode layer 2 having the conductive elastomer layer 20.

The wearable bioelectrode 100 can make one surface 22 of the conductive elastomer layer 20 follow the body of the subject, and can detect a bioelectric signal generated from biological activity such as a heart, muscle, skin, and a nerve. In a case where the wearable bioelectrode 100 includes a plurality of conductive elastomer layers 20 in an in-plane direction of the flexible base material 10, the wearable bioelectrode 100 can be suitably used as an electrode for measuring an electrocardiogram.

Examples of the subject include a human and an animal other than a human.

The wearable bioelectrode 100 can be used as a dry sensor that is simple and can be repeatedly used, instead of a wet sensor that requires application of a gel to a measuring portion of a subject.

The flexible base material 10 has an electrode forming region where the conductive elastomer layer 20 is formed in a top view. In the electrode forming region, the flexible base material 10 closely adheres to the conductive elastomer layer 20 in a case of non-stretching and non-contraction or stretching and contraction, and the mechanical strength of the entire wearable bioelectrode 100 can be increased.

In addition, the flexible base material 10 may have an electrode non-forming region where the conductive elastomer layer 20 is not formed in the periphery of the electrical forming region. A mounting part such as a sewing thread or a snap button for being placed on clothing or the like can be attached to the electrode non-forming region. Alternatively, in a case where the wearable bioelectrode 100 is wound around a body, such as a wrist or an ankle, portions in the electrode non-forming region of the flexible base material 10 may be overlapped in the thickness direction and fixed to the body.

As an example of the flexible base material 10, at least a part of the flexible base material 10, for example, a main body 12 has a band shape that can be wound around a body as shown in Fig. 3. Such a wearable bioelectrode 100 has a suitable structure for a band-mounted bioelectrode that can be wound around a part of the body, such as a wrist or an ankle.

Further, the flexible base material 10 having a band shape in Fig. 3 may include the main body 12 and an extension portion 14 that protrudes in the in-plane direction of the main body 12. Even in a case where the main body 12 is deformed into a ring structure or the like, the deformation of the extension portion 14 is suppressed. Therefore, occurrence of connection failure in an external connection portion 30 attached to the extension portion 14 can be suppressed.

The conductive elastomer layer 20 is formed in a sheet shape and has an exposed surface that is brought into direct contact with the body.

In the present specification, in a case where the thickness of the conductive elastomer layer 20 is defined as D (mm) and the area of one surface 22 of the conductive elastomer layer 20 in a top view is defined as S (mm²), S and D of the sheet shape satisfy, for example, 50 ≤ S/D, preferably 200 ≤ S/D, and more preferably 400 ≤ S/D. The upper limit of S/D may be set according to the measurement site of a subject, and is not particularly limited, and may be, for example, S/D ≤ 10⁷.

The conductive elastomer layer 20 is in contact with the body during the use of the wearable bioelectrode 100 and can closely follow the contact surface along the surface shape of the body or deformation of the body.

The external part of the wearable bioelectrode 100 may be formed such that the conductive elastomer layer 20 is connected thereto, but may be formed such that a connection portion 26, which is another member electrically connected to the conductive elastomer layer 20, is connected thereto. As shown in Fig. 3, the connection portion 26 may be formed to be electrically connected to the conductive elastomer layer 20 via an elastic wiring 24.

The conductive elastomer layer 20, the elastic wiring 24, and the connection portion 26 are formed of conductive elastomers which are the same as or different from each other and preferably conductive elastomers which are the same as each other.

Each part constituting the conductive elastomer layer 20 may be formed of a printing layer formed by a printing method using a conductive paste. In this case, each part including the conductive elastomer layer 20, the elastic wiring 24, and the connection portion 26 may be seamlessly formed.

The external connection portion 30 can transmit the bioelectric signal detected through the conductive elastomer layer 20 to the outside, such as an electronic component.

The wearable bioelectrode 100 of Fig. 3 includes the external connection portion 30.

The external connection portion 30 is provided over the flexible base material 10 on a side opposite to the conductive elastomer layer 20. For example, the external connection portion 30 of Fig. 3 is formed on the other surface 13 of the flexible base material 10. In this manner, it is possible to prevent the external connection portion 30 from coming into contact with the body together with the conductive elastomer layer 20 during the use of the wearable bioelectrode 100 and to suppress the interference with detection of the bioelectric signal.

The external connection portion 30 is not particularly limited as long as the external connection portion 30 is formed to be electrically connected to the conductive elastomer layer 20, and is formed of a conductive material such as a metal material or a conductive elastomer.

The shape of the external connection portion 30 is not particularly limited, and the external connection portion 30 may have a connector to which an electronic component can be connected and a structure to which a wiring is easily attached.

According to an example, the external connection portion 30 may be formed of a metal snap button.

The external connection portion 30 of Fig. 4 is a male snap button, and is fixed to the flexible base material 10 by using an attachment plate 32 and an attachment pin 34, which are attachment tools made of a metal. A shift in position of the fixed position of the external connection portion 30 can be suppressed by a fixing method of sandwiching the flexible base material 10 between the external connection portion 30 and the attachment plate 32.

The attachment pin 34 can be brought into contact with a part of the conductive elastomer layer 20, for example, the connection portion 26, to achieve conduction between the conductive elastomer layer 20 and the external connection portion 30.

In addition, a part or the entire surface of the attachment plate 32 in Fig. 4 is covered with the insulating protective layer 52. The insulating protective layer 52 can prevent the contact between the attachment plate 32 and the body. The insulating protective layer 52 may be formed of an insulating material, and may be formed of, for example, an insulating elastomer such as silicone rubber.

A biosensor according to the present embodiment will be described.

The biosensor includes only one or two or more wearable bioelectrodes 100 depending on the applications thereof. The biosensor may be directly placed on the body, or may be placed in a body attachment member such as clothing.

The biosensor includes an electronic component that can be electrically connected to the wearable bioelectrode 100 via the external connection portion 30.

As the electronic component, known components can be used according to the various applications, and examples of the electronic component include an amplifier, an AD converter, a CPU, a memory, a communication circuit, a wireless communication unit, an analog filter, a capacitor, a resistor, and a battery. Among the examples, one or two or more components may be modularized on a circuit board. In this manner, the biosensor can be used as a wearable device.

In addition, as the electronic component, another sensor such as an acceleration sensor, a temperature sensor, or a pressure sensor may be used in combination.

Fig. 5 is a view for describing an example of the configuration of the biosensor 200.

The biosensor 200 in Fig. 5 includes the wearable bioelectrode 100, a connector 210, a cable 220, a sensor module 230, and a computer 240.

The connector 210 has a structure that can be electrically coupled to the external connection portion 30 of the wearable bioelectrode 100, and may have, for example, a female snap button.

The cable 220 electrically connects the connector 210, the sensor module 230 serving as an electronic component, and the computer 240 to each other.

In a case of measuring the electrocardiogram, an ECG sensor module can be used as the sensor module 230. The sensor module 230 has an electric circuit that suppresses interference from an external RF source, a line frequency, and electric noise, and can suppress noise during the measurement of the bioelectric signal.

The computer 240 can acquire the bioelectric signal and can generate and output a waveform of a biological potential such as a surface electromyogram, an electrocardiogram, a skin potential, and a brain wave. A single board computer formed of a printed substrate including a CPU, a peripheral component, an input/output interface, a connector, and the like may be used as the computer 240.

A biological signal measurement system according to the present embodiment will be described.

The biological signal measurement system includes a biosensor. The biological signal measurement system may be a system (measurement device) that displays, analyzes, or stores data received from the biosensor.

Hereinafter, the materials of the conductive elastomer layer 20 and the insulating elastomer layer 60 will be described.

The conductive elastomer layers 20, 20a, and 20b in the flexible sheet electrode 1 may each contain a conductive elastomer.

The insulating elastomer layers 60, 60a, and 60b in the flexible sheet electrode 1 may each contain an insulating elastomer.

The insulating elastomer and the conductive elastomer may contain elastomer materials that are the same as or different from each other.

As the insulating elastomer, for example, silicone rubber, urethane rubber, fluororubber, nitrile rubber, acrylic rubber, styrene rubber, chloroprene rubber, or ethylene propylene rubber can be used. Among these, the elastomer includes one or more thermosetting elastomers (elastomer materials) selected from the group consisting of silicone rubber, urethane rubber, and fluororubber.

The insulating elastomer may be formed of only the elastomer material, or may be formed to contain the elastomer material and a non-conductive filler. According to an example, the insulating elastomer contains silicone rubber and preferably silicone rubber and a non-conductive filler. The silicone rubber is chemically stable and also has excellent mechanical strength among elastomers.

Among these, from the viewpoint of hygiene, it is preferable that silicone rubber having high biocompatibility is used as the elastomer material.

As the conductive elastomer, for example, silicone rubber, urethane rubber, fluororubber, nitrile rubber, acrylic rubber, styrene rubber, chloroprene rubber, ethylene propylene rubber, or the like can be used. Among these, the elastomer contains one or more kinds of thermosetting elastomers (elastomer materials) selected from the group consisting of silicone rubber, urethane rubber, and fluororubber, and a conductive filler.

Preferred examples of the conductive elastomer include silicone rubber and a conductive filler. In this manner, the elasticity and the conductivity of the conductive elastomer can be increased.

At least one and preferably both of the insulating elastomer and the conductive elastomer may contain a non-conductive filler. A known material can be used as the non-conductive filler, and for example, silica particles, silicone rubber particles, or talc may be used. Among these, the non-conductive filler may include silica particles.

The conductive filler may include one or two or more selected from the group consisting of a powdery or fibrous metal-based filler, a carbon-based filler, a metal oxide filler, and a metal-plated filler. Among these, a metal-based filler such as silver powder may be used as the conductive filler.

The conductive elastomer may further include a non-conductive filler in addition to the conductive filler. In this manner, the mechanical characteristics of the conductive elastomer can be enhanced.

As a specific example, the conductive elastomer layer 20 may contain silver powder as the conductive filler and preferably contain scale-like silver powder. In this manner, the conductivity and elastic conductivity can be further improved.

Further, the conductive elastomer layer 20 may contain silver powder and silica particles as the non-conductive filler. In this manner, the elastic durability of the conductive elastomer layer 20 can be improved.

At least two or all of the conductive elastomer of the conductive elastomer layer 20 and the insulating elastomer of the insulating elastomer layer 60 may contain the same elastomer material.

In the present specification, the expression "containing the same elastomer material" denotes that the elastomer contains at least one or more of the same kinds of elastomer materials among the kinds of thermosetting elastomers described above.

Further, in a case where the elastomer contains the same silicone rubber, the silicone rubber may be formed of a cured product of a silicone rubber-based curable composition containing a vinyl group-containing organopolysiloxane.

From the viewpoint of mechanical strength or the like, it is preferable that the insulating elastomer layer 60 and/or the conductive elastomer layer 20 contain silicone rubber.

The insulating elastomer may be formed of a cured product of a silicone rubber-based curable composition containing a vinyl group-containing organopolysiloxane. In addition, the conductive elastomer may be formed of a conductive filler and a cured product of a silicone rubber-based curable composition containing a vinyl group-containing organopolysiloxane.

Hereinafter, components of the silicone rubber-based curable composition will be described in detail.

In the present specification, the expression "containing the same silicone rubber" denotes that the silicone rubber-based curable composition contains at least the same kind of vinyl group-containing linear organopolysiloxane, and may further contain one or two or more selected from the group consisting of the same kind of crosslinking agent, the same kind of non-conductive filler, the same kind of silane coupling agent, and the same kind of catalyst.

The same type of vinyl group-containing linear organopolysiloxanes may contain at least vinyl groups having the same functional groups and may be linear. The amount of vinyl groups in the molecule, the molecular weight distribution, or the addition amounts thereof may vary between the vinyl group-containing linear organopolysiloxanes.

The same type of crosslinking agents just need to have at least a common structure such as a linear structure or a branched structure. The crosslinking agents may contain functional groups with different molecular weight distributions in the molecule, and the addition amounts thereof may be different.

The same type of non-conductive fillers just need to have at least a common constituent material, and the particle diameter, specific surface area, surface treatment agent, or the addition amount thereof may vary between the fillers.

The same type of silane coupling agents just need to have at least a common functional group and may have other functional groups in the molecule, and the addition amounts thereof may be different.

The same type of catalysts just need to have at least a common constituent material. Different compositions may be contained in the catalysts, or the addition amounts thereof may vary between the catalysts.

The silicone rubber-based curable composition constituting the same silicone rubber may further contain one or two or more selected from the group consisting of a vinyl group-containing linear organopolysiloxane, a crosslinking agent, a non-conductive filler, a silane coupling agent, and a catalyst of different kinds.

The silicone rubber-based curable composition of the present embodiment is capable of containing a vinyl group-containing organopolysiloxane (A). The vinyl group-containing organopolysiloxane (A) is a polymer containing the silicone rubber-based curable composition according to the present embodiment as a main element.

The vinyl group-containing organopolysiloxane (A) can contain a vinyl group-containing linear organopolysiloxane (A1) having a linear structure.

The vinyl group-containing linear organopolysiloxane (A1) has a linear structure and contains a vinyl group which functions as a crosslinking point during curing.

The content of the vinyl group in the vinyl group-containing linear organopolysiloxane (A1) is not particularly limited, but it is preferable that, for example, the vinyl group-containing linear organopolysiloxane contains two or more vinyl groups in a molecule and the content thereof is less than or equal to 15% by mole. In a case where the content of the vinyl group is in the above range, the amount of the vinyl group in the vinyl group-containing linear organopolysiloxane (A1) can be optimized, and a network between the respective components, which will be described later, can be reliably formed.

In the present specification, a term "to" is used to indicate that an upper limit and a lower limit are included in a range unless otherwise specified.

In the present specification, the vinyl group content is mol% of a vinyl group-containing siloxane unit with respect to 100 mol% of all the units forming the vinyl group-containing linear organopolysiloxane (A1). Here, it is assumed that there may be one vinyl group for each vinyl group-containing siloxane unit.

The polymerization degree of the vinyl group-containing linear organopolysiloxane (A1) is not particularly limited and is, for example, preferably in a range of about 1,000 to 10,000 and more preferably in a range of about 2,000 to 5,000. The polymerization degree can be determined, for example, as a polystyrene-equivalent number-average polymerization degree (or number-average molecular weight) or the like gel permeation chromatography (GPC) using chloroform as an elution solvent.

The specific gravity of the vinyl group-containing linear organopolysiloxane (A1) is not particularly limited and is preferably in a range of about 0.9 to 1.1

Using the vinyl group-containing linear organopolysiloxane (A1) having the polymerization degree and the specific gravity in the ranges described above makes it possible to obtain silicone rubber having higher heat resistance, higher flame retardancy, higher chemical stability, and the like.

It is preferable that the vinyl group-containing linear organopolysiloxane (A1) have a structure represented by the following Formula (1).

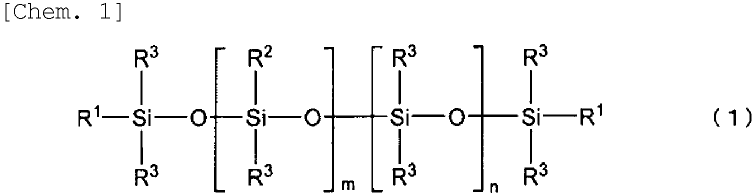

In Formula (1), R¹ represents a substituted or unsubstituted alkyl group, alkenyl group, or aryl group having 1 to 10 carbon atoms, or a hydrocarbon group as a combination of these. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, a butenyl group, and the like. Among these, a vinyl group is preferable. Examples of the aryl group having 1 to 10 carbon atoms include a phenyl group and the like.

R² represents a substituted or unsubstituted alkyl group, alkenyl group, or aryl group having 1 to 10 carbon atoms, or a hydrocarbon group as a combination of these. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

R³ represents a substituted or unsubstituted alkyl group or aryl group having 1 to 8 carbon atoms, or a hydrocarbon group as a combination of these. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group.

Examples of substituents of R¹ and R² in Formula (1) include a methyl group, a vinyl group, and the like. Examples of substituents of R³ include a methyl group and the like.

In Formula (1), a plurality of R¹'s is independent of each other, and may be the same as or different from each other. The same shall be applied to R² and R³.

m and n each represent the number of repeating units configuring the vinyl group-containing linear organopolysiloxane (A1) represented by Formula (1). m represents an integer of 0 to 2,000, and n represents an integer of 1,000 to 10,000. m is preferably 0 to 1,000, and n is preferably 2,000 to 5,000.

Examples of specific structures of the vinyl group-containing linear organopolysiloxane (A1) represented by Formula (1) include a structure represented by the following Formula (1-1).

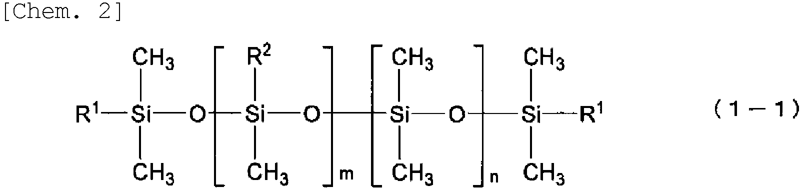

In Formula (1-1), R¹ and R² each independently represent a methyl group or a vinyl group, and at least one of R¹ or R² represents a vinyl group.

The vinyl group-containing linear organopolysiloxane (A1) may include a first vinyl group-containing linear organopolysiloxane (A1-1) which contains two or more vinyl groups in a molecule and in which the content thereof is less than or equal to 0.4% by mole. The amount of the vinyl group in the first vinyl group-containing linear organopolysiloxane (A1-1) may be less than or equal to 0.1% by mole.

In addition, the vinyl group-containing linear organopolysiloxane (A1) may include the first vinyl group-containing linear organopolysiloxane (A1-1) and a second vinyl group-containing linear organopolysiloxane (A1-2) in which the content of the vinyl group is in a range of 0.5% to 15% by mole.

By using a combination of the first vinyl group-containing linear organopolysiloxane (A1-1) and the second vinyl group-containing linear organopolysiloxane (A1-2) in which the content of the vinyl group is high, as raw rubber which is a raw material of silicone rubber, the vinyl groups can be unevenly distributed, and coarseness and fineness of the crosslinking density can be more effectively formed in the crosslinked network of the silicone rubber. As a result, the tear strength of the silicone rubber can be more effectively improved.

Specifically, as the vinyl group-containing linear organopolysiloxane (A1), for example, the first vinyl group-containing linear organopolysiloxane (A1-1) having two or more of a unit in which R¹ in Formula (1-1) represents a vinyl group and/or a unit in which R² in Formula (1-1) represents a vinyl group in a molecule and the content thereof is 0.4% by mole or lower or the second vinyl group-containing linear organopolysiloxane (A1-2) having 0.5% to 15% by mole of a unit in which R¹ in Formula (1-1) represents a vinyl group and/or a unit in which R² in Formula (1-1) represents a vinyl group is preferably used.

The first vinyl group-containing linear organopolysiloxane (A1-1) preferably has a vinyl group content of 0.01% to 0.2% by mole. In addition, the second vinyl group-containing linear organopolysiloxane (A1-2) preferably has a vinyl group content of 0.8% to 12% by mole.

In a case where the first vinyl group-containing linear organopolysiloxane (A1-1) and the second vinyl group-containing linear organopolysiloxane (A1-2) are mixed together, the ratio between (A1-1) and (A1-2) is not particularly limited. For example, the weight ratio (A1-1): (A1-2) is preferably 50:50 to 95:5, and more preferably 80:20 to 90:10.

As each of the first and second vinyl group-containing linear organopolysiloxanes (A1-1) and (A1-2), only one compound may be used, or two or more compounds may be used in combination.

The vinyl group-containing organopolysiloxane (A) may contain a vinyl group-containing branched organopolysiloxane (A2) having a branched structure.

### <<Organohydrogen polysiloxane (B)>>

The silicone rubber-based curable composition according to the present embodiment may include an organohydrogen polysiloxane (B) .

The organohydrogen polysiloxane (B) is classified into a linear organohydrogen polysiloxane (B1) having a linear structure and a branched organohydrogen polysiloxane (B2) having a branched structure, and can include either or both of these.

The linear organohydrogen polysiloxane (B1) is a polymer that has a linear structure and a structure (=Si-H) in which hydrogen is directly bonded to Si, and has a hydrosilylation reaction with vinyl groups contained in the components mixed with the silicone rubber-based curable composition in addition to the vinyl groups of the vinyl group-containing organopolysiloxane (A) to crosslink the components.

The molecular weight of the linear organohydrogen polysiloxane (B1) is not particularly limited. For example, the weight-average molecular weight thereof is preferably less than or equal to 20,000 and more preferably greater than or equal to 1,000 and less than or equal to 10,000.

The weight-average molecular weight of the linear organohydrogen polysiloxane (B1) can be measured as a polystyrene-equivalent value by gel permeation chromatography (GPC) using chloroform as an elution solvent.

It is preferable that the linear organohydrogen polysiloxane (B1) do not have a vinyl group in general. In a case where the linear organohydrogen polysiloxane (B1) does not have a vinyl group, it is possible to reliably prevent the occurrence of a crosslinking reaction in the molecule of the linear organohydrogen polysiloxane (B1).

As the linear organohydrogen polysiloxane (B1), for example, a compound having a structure represented by the following Formula (2) is preferably used.

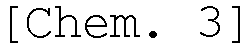

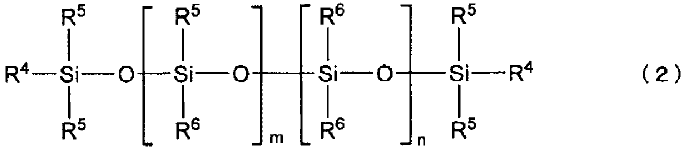

In Formula (2), R⁴ represents a substituted or unsubstituted alkyl group, alkenyl group, or aryl group having 1 to 10 carbon atoms, a hydrocarbon group as a combination of these, or a hydride group. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, a butenyl group, and the like. Examples of the aryl group having 1 to 10 carbon atoms include a phenyl group.

R⁵ represents a substituted or unsubstituted alkyl group, alkenyl group, or aryl group having 1 to 10 carbon atoms, a hydrocarbon group as a combination of these, or a hydride group. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, a butenyl group, and the like. Examples of the aryl group having 1 to 10 carbon atoms include a phenyl group.

In Formula (2), a plurality of R⁴'s is independent of each other, and may be the same as or different from each other. The same shall be applied to R⁵. Here, at least two or more among the plurality of R⁴'s and the plurality of R⁵'s represent hydride groups.

R⁶ represents a substituted or unsubstituted alkyl group or aryl group having 1 to 8 carbon atoms, or a hydrocarbon group as a combination of these. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group. A plurality of R⁶'s is independent of each other, and may be the same as or different from each other.

Examples of substituents of R⁴, R⁵, and R⁶ in Formula (2) include a methyl group, a vinyl group, and the like. From the viewpoint of preventing an intramolecular crosslinking reaction, a methyl group is preferable.

m and n each independently represent the number of repeating units configuring the linear organohydrogen polysiloxane (B1) represented by Formula (2), m represents an integer of 2 to 150, and n represents an integer of 2 to 150. It is preferable that m represent an integer of 2 to 100 and n represent an integer of 2 to 100.

As the linear organohydrogen polysiloxane (B1), only one compound may be used alone, or two or more compounds may be used in combination.

Having a branched structure, the branched organohydrogen polysiloxane (B2) is a component that largely contributes to the formation of a density-varying crosslinking structure in the silicone rubber system by forming a region having a high crosslinking density. In addition, just as the linear organohydrogen polysiloxane (B1), the branched organohydrogen polysiloxane (B2) is a polymer that has a structure (=Si-H) in which hydrogen is directly bonded to Si, and has a hydrosilylation reaction with vinyl groups of components mixed with the silicone rubber-based curable composition in addition to the vinyl groups of the vinyl group-containing organopolysiloxane (A) to crosslink these components.

The specific gravity of the branched organohydrogen polysiloxane (B2) is in a range of 0.9 to 0.95.

It is preferable that the branched organohydrogen polysiloxane (B2) do not have a vinyl group in general. In a case where the branched organohydrogen polysiloxane (B2) does not have a vinyl group, it is possible to reliably prevent the occurrence of a crosslinking reaction in the molecule of the linear organohydrogen polysiloxane (B2).

The branched organohydrogen polysiloxane (B2) is preferably represented by the following Average Compositional Formula (c).

Average Compositional Formula (c) (Hₐ(R⁷)₃₋ₐSiO_{1/2})ₘ(SiO_{4/2})ₙ

In Formula (c), R⁷ represents a monovalent organic group, a represents an integer of 1 to 3, m represents the number of Hₐ(R⁷)₃₋ₐSiO_{1/2} units, and n represents the number of SiO_{4/2} units.

In Formula (c), R⁷ represents a monovalent organic group, and preferably represents a substituted or unsubstituted alkyl group or aryl group having 1 to 10 carbon atoms, or a hydrocarbon group as a combination of these. Examples of the alkyl group having 1 to 10 carbon atoms include a methyl group, an ethyl group, a propyl group, and the like. Among these, a methyl group is preferable. Examples of the aryl group having 1 to 10 carbon atoms include a phenyl group.

In Formula (c), a represents the number of hydride groups (hydrogen atoms directly bonded to Si) which is an integer of 1 to 3. a is preferably 1.

In addition, in Formula (c), m represents the number of Hₐ(R⁷)₃₋ₐSiO_{1/2} units, and n represents the number of SiO_{4/2} units.

The branched organohydrogen polysiloxane (B2) has a branched structure. The difference between the linear organohydrogen polysiloxane (B1) and the branched organohydrogen polysiloxane (B2) is whether the structure thereof is linear or branched. Assuming that the number of Si is 1, the number of alkyl groups R bonded to Si is in a range of 1.8 to 2.1 in the linear organohydrogen polysiloxane (B1) and is in a range of 0.8 to 1.7 in the branched organohydrogen polysiloxane (B2).

The residual amount of the branched organohydrogen polysiloxane (B2) is greater than or equal to 5% due to having a branched structure in a case of being heated, for example, to 1,000°C at a heating rate of 10 °C/min in a nitrogen atmosphere. On the other hand, having a linear structure, the linear organohydrogen polysiloxane (B1) substantially does not leave residues in a case where the compound is heated under the above conditions.

Specific examples of the branched organohydrogen polysiloxane (B2) include compounds having a structure represented by the following Formula (3).

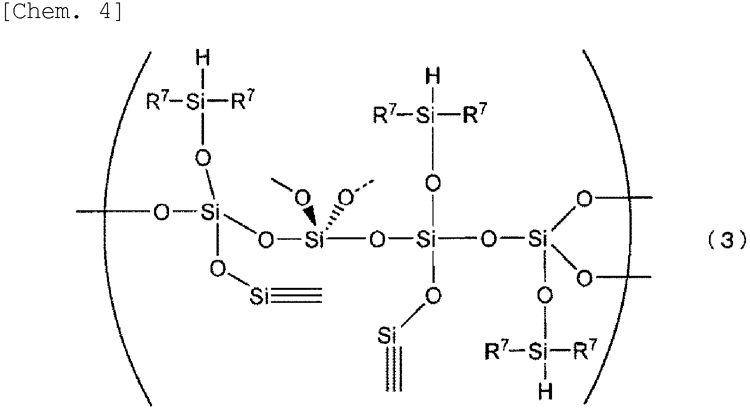

In Formula (3), R⁷ represents a substituted or unsubstituted alkyl group or aryl group having 1 to 8 carbon atoms, a hydrocarbon group as a combination of these, or a hydrogen atom. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group. Examples of substituents of R⁷ include a methyl group and the like.

In Formula (3), a plurality of R⁷'s is independent of each other, and may be the same as or different from each other.

In Formula (3), "-O-Si=" means that Si has a branched structure that expands three-dimensionally.

As the branched organohydrogen polysiloxane (B2), only one compound may be used alone, or two or more compounds may be used in combination.

In each of the linear organohydrogen polysiloxane (B1) and the branched organohydrogen polysiloxane (B2), the amount of hydrogen atoms (hydride groups) directly bonded to Si is not particularly limited. Here, in the silicone rubber-based curable composition, the total amount of hydride groups in the linear organohydrogen polysiloxane (B1) and the branched organohydrogen polysiloxane (B2) is preferably 0.5 to 5 mol and more preferably 1 to 3.5 mol with respect to 1 mol of vinyl groups in the vinyl group-containing linear organopolysiloxane (A1). In a case where the total amount of hydride groups is in the above range, a crosslinked network can be reliably formed among the linear organohydrogen polysiloxane (B1) and the branched organohydrogen polysiloxane (B2), and the vinyl group-containing linear organopolysiloxane (A1).

### «Silica particles (C)>>

The silicone rubber-based curable composition according to the present embodiment can contain silica particles (C) as a non-conductive filler, as necessary.

The silica particles (C) are not particularly limited. For example, fumed silica, sintered silica, precipitated silica, or the like is used. These may be used alone or in combination of two or more kinds thereof.

The specific surface area of the silica particles (C) measured using, for example, a BET method is, for example, preferably 50 to 400 m²/g, and more preferably 100 to 400 m²/g. The average primary particle diameter of the silica particles (C) is, for example, preferably 1 to 100 nm and more preferably about 5 to 20 nm.

Using the silica particles (C) having the specific surface area and the average particle diameter in the above ranges makes it possible to improve the hardness and the mechanical strength of the formed silicone rubber and, particularly, to improve the tensile strength of the formed silicone rubber.

### <<Silane coupling agent (D)>

The silicone rubber-based curable composition according to the present embodiment may contain a silane coupling agent (D).

The silane coupling agent (D) may have a hydrolyzable group. The hydrolyzable group is hydrolyzed into a hydroxyl group by water. The hydroxyl group has a dehydration condensation reaction with a hydroxyl group on the surface of the silica particles (C), which can modify the surface of the silica particles (C).

The silane coupling agent (D) can include a silane coupling agent having a hydrophobic group. Thus, since the hydrophobic group is provided on a surface of the silica particles (C), the aggregation force of the silica particles (C) is reduced (aggregation by hydrogen bonding due to silanol groups is reduced) in the silicone rubber-based curable composition and also in the silicone rubber, and as a result, it is presumed that the dispersibility of the silica particles in the silicone rubber-based curable composition is improved. Thus, an interface between the silica particles and the rubber matrix is increased and the reinforcing effect of the silica particles is enhanced. Further, it is presumed that the slipperiness of the silica particles in the matrix is improved in a case of matrix modification of the rubber. Further, the improvement of dispersibility and slipperiness of the silica particles (C) leads to the improvement of the mechanical strength (for example, tensile strength or tear strength) of the silicone rubber by the silica particles (C).

The silane coupling agent (D) may include a silane coupling agent having a vinyl group. In a case where the silane coupling agent has a vinyl group, the vinyl group is introduced into the surface of the silica particles (C). Therefore, in a case where the silicone rubber-based curable composition is cured, that is, in a case where the vinyl groups of the vinyl group-containing organopolysiloxane (A) and the hydride groups in the organohydrogen polysiloxane (B) have a hydrosilylation reaction to form a network (crosslinked structure), the vinyl groups of the silica particles (C) are also involved in the hydrosilylation reaction with the hydride groups of the organohydrogen polysiloxane (B). As a result, the silica particles (C) are also incorporated into the network. Accordingly, it is possible to achieve the reduction in hardness and the increase in modulus of the formed silicone rubber.

As the silane coupling agent (D), a silane coupling agent having a hydrophobic group and a silane coupling agent having a vinyl group can be used in combination.

Examples of the silane coupling agent (D) include a compound represented by the following Formula (4).

Yₙ-Si-(X)₄₋ₙ (4)

In Formula (4), n represents an integer of 1 to 3. Y represents any functional group among a hydrophobic group, a hydrophilic group, and a vinyl group. When n represents 1, Y represents a hydrophobic group. When n represents 2 or 3, at least one of Y's represents a hydrophobic group. X represents a hydrolyzable group.

The hydrophobic group is an alkyl group or aryl group having 1 to 6 carbon atoms, or a hydrocarbon group as a combination of these. Examples of the hydrophobic group include a methyl group, an ethyl group, a propyl group, a phenyl group, and the like. Among these, a methyl group is particularly preferable.

Examples of the hydrophilic group include a hydroxyl group, a sulfonate group, a carboxyl group, a carbonyl group, and the like. Among these, a hydroxyl group is particularly preferable. The hydrophilic group may be incorporated into the silane coupling agent as a functional group. From the viewpoint of making the silane coupling agent (D) hydrophobic, it is preferable that the silane coupling agent (D) do not contain a hydrophilic group.

Examples of the hydrolyzable group include an alkoxy group such as a methoxy group or an ethoxy group, a chloro group, a silazane group, and the like. Among these, in view of high reactivity with the silica particles (C), a silazane group is preferable. Owing to its structural characteristics, the silane coupling agent having a silazane group as a hydrolyzable group has two structures represented by (Yₙ-Si-) in Formula (4).

Specific examples of the silane coupling agent (D) represented by Formula (4) include an agent having a hydrophobic group as a functional group, such as alkoxysilanes such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, and decyltrimethoxysilane, chlorosilanes such as methyltrichlorosilane, dimethyldichlorosilane, trimethylchlorosilane, and phenyltrichlorosilane, and hexamethyldisilazane; an agent having a vinyl group as a functional group, such as alkoxysilanes such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyltrimethoxysilane, and vinylmethyldimethoxysilane, chlorosilanes such as vinyltrichlorosilane and vinylmethyldichlorosilane, and divinyltetramethyldisilazane. Among them, hexamethyldisilazane having a hydrophobic group, and divinyltetramethyldisilazane having a vinyl group are particularly preferable when the above description is taken into consideration.

In the present embodiment, the lower limit of the content of the silane coupling agent (D) with respect to the total amount of 100 parts by weight of the vinyl group-containing organopolysiloxane (A) is preferably greater than or equal to 1% by mass, more preferably greater than or equal to 3% by mass, and still more preferably greater than or equal to 5% by mass. Furthermore, the upper limit of the content of the silane coupling agent (D) with respect to the total amount of 100 parts by weight of the vinyl group-containing organopolysiloxane (A) is preferably less than or equal to 100% by mass, more preferably less than or equal to 80% by mass, and still more preferably less than or equal to 40% by mass.

In a case where the content of the silane coupling agent (D) is set to be greater than or equal to the above-described lower limits and in a case where the silica particles (C) are used, the mechanical strength of the entire silicone rubber can be improved. Furthermore, setting the content of the silane coupling agent (D) to be less than or equal to the aforementioned upper limit makes it possible to obtain silicone rubber having appropriate mechanical characteristics.

### <<Platinum or platinum compound (E)>>

The silicone rubber-based curable composition of the present embodiment is capable of containing platinum or a platinum compound (E).

The platinum or platinum compound (E) is a catalyst component that functions as a catalyst during curing. The addition amount of the platinum or platinum compound (E) is the amount of the catalyst.

As the platinum or platinum compound (E), known platinum or platinum compounds can be used. Examples thereof include platinum black, silica or carbon black on which platinum is supported, platinic chloride or an alcohol solution of platinic chloride, a complex salt of platinic chloride and olefin, a complex salt of platinic chloride and vinylsilxoane, and the like.

As the platinum or platinum compound (E), only one platinum element or one platinum compound may be used alone, or two or more platinum elements or two platinum compounds may be used in combination.

### <<Water (F)>>

In addition, the silicone rubber-based curable composition of the present embodiment may include, in addition to the components (A) to (E), water (F).

The water (F) is a component that functions as a dispersion medium for dispersing the respective components in the silicone rubber-based curable composition and contributes to the reaction between the silica particles (C) and the silane coupling agent (D). Therefore, the silica particles (C) and the silane coupling agent (D) can be more reliably linked to each other in the silicone rubber, and the silicone rubber can show uniform characteristics as a whole.

Furthermore, in a case where water (F) is contained, a content thereof can be appropriately set, but is specifically, for example, preferably in the range of 10 to 100 parts by weight, and more preferably in the range of 30 to 70 parts by weight, with respect to 100 parts by weight of the silane coupling agent (D). Thus, the progress of a reaction between the silane coupling agent (D) and the silica particles (C) can be more reliably made.

### (Other components)

Further, the silicone rubber-based curable composition according to the present embodiment may further contain other components in addition to the components (A) to (F). Examples of those other components include an inorganic filler other than the silica particles (C), such as diatomaceous earth, iron oxide, zinc oxide, titanium oxide, barium oxide, magnesium oxide, cerium oxide, calcium carbonate, magnesium carbonate, zinc carbonate, glass wool, or mica, and an additive such as a reaction inhibitor, a dispersant, a pigment, a dye, an antistatic agent, an antioxidant, a flame retardant, or a thermal conductivity enhancing agent.

In addition, the content proportion of each of the components in the silicone rubber-based curable composition is not particularly limited, but is set in the following manner, for example.

In the present embodiment, the upper limit of the content of the silica particles (C) may be, for example, less than or equal to 60 parts by weight, preferably less than or equal to 50 parts by weight, and more preferably less than or equal to 40 parts by weight with respect to 100 parts by weight of the total amount of the vinyl group-containing organopolysiloxane (A). Accordingly, it is possible to realize the balance with mechanical strength such as hardness or tensile strength. Further, the lower limit of the content of the silica particles (C) is not particularly limited, but may be, for example, greater than or equal to 10 parts by weight with respect to 100 parts by weight of the total amount of the vinyl group-containing organopolysiloxane (A).

The silane coupling agent (D) is contained, for example, in a proportion of preferably greater than or equal to 5 parts by weight and less than or equal to 100 parts by weight, more preferably in a proportion of greater than or equal to 5 parts by weight and less than or equal to 40 parts by weight, with respect to 100 parts by weight of the vinyl group-containing organopolysiloxane (A). Thus, the dispersibility of the silica particles (C) in the silicone rubber-based curable composition can be reliably improved.

The content of the organohydrogen polysiloxane (B) is, for example, preferably greater than or equal to 0.5 parts by weight and less than or equal to 20 parts by weight and more preferably greater than or equal to 0.8 parts by weight and less than or equal to 15 parts by weight with respect to 100 parts by weight of the total amount of the vinyl group-containing organopolysiloxane (A), the silica particles (C), and the silane coupling agent (D). In a case where the content of (B) is within the range, there is a possibility that a more effective curing reaction proceeds.

A content of platinum or the platinum compound (E) means a catalytic amount and can be set appropriately. Specifically, a content of platinum or the platinum compound (E) is such that platinum group metals in this component are 0.01 to 1,000 ppm a unit of weight, and are preferably 0.1 to 500 ppm in a unit of weight, with respect to a total amount of the vinyl group-containing organopolysiloxane (A), the silica particles (C), and the silane coupling agent (D). In a case where the content of platinum or the platinum compound (E) is set to be greater than or equal to the above-described lower limits, the silicone rubber composition to be obtained can be sufficiently cured. In a case where the content of platinum or the platinum compound (E) is set to be less than or equal to the above-described upper limits, the curing rate of the silicone rubber composition to be obtained can be improved.

Furthermore, in a case where water (F) is contained, a content thereof can be appropriately set, but is specifically, for example, preferably in the range of 10 to 100 parts by weight, and more preferably in the range of 30 to 70 parts by weight, with respect to 100 parts by weight of the silane coupling agent (D). Thus, the progress of a reaction between the silane coupling agent (D) and the silica particles (C) can be more reliably made.

In the present embodiment, the hardness, the tensile strength, the breaking elongation, and the tear strength can be controlled, for example, by appropriately selecting the kind and the blending amount of each component contained in the silicone rubber-based curable composition, a method of preparing the silicone rubber-based curable composition, and the like. Among these, examples of the factors for setting the hardness, the tensile strength, the breaking elongation, and the tear strength to be in desirable numerical ranges include appropriately controlling the kind and the blending ratio of the resin constituting the silicone rubber, and the crosslinking density, the crosslinking structure, and the like of the resin; improving the blending ratio of the inorganic filler and the bonding of the inorganic filler to the rubber; using the first vinyl group-containing linear organopolysiloxane (A1-1) in which the amount of the vinyl group is less than or equal to 0.4% by mole; using the silane coupling agent containing a vinyl group; and appropriately adjusting the content of the silica particles (C) and the content of the silane coupling agent.

### <Method for producing silicone rubber>

Next, the method for producing a silicone rubber of the present embodiment will be described.

In the method for producing a silicone rubber of the present embodiment, a silicone rubber can be obtained by preparing a silicone rubber-based curable composition and curing the silicone rubber-based curable composition.

The details thereof will be described below.

First, the respective components of the silicone rubber-based curable composition are uniformly mixed by any kneading device to prepare a silicone rubber-based curable composition.
[1] For example, predetermined amounts of the vinyl group-containing organopolysiloxane (A), the silica particles (C), and the silane coupling agent (D) are weighed and kneaded using a kneading device, thereby obtaining a kneaded product containing each of these components (A), (C), and (D).

Furthermore, the kneaded product is preferably obtained by kneading the vinyl group-containing organopolysiloxane (A) and the silane coupling agent (D) in advance, and then kneading (mixing) the silica particles (C) therewith. Thus, the dispersibility of the silica particles (C) in the vinyl group-containing organopolysiloxane (A) is further improved.

In addition, in a case of obtaining the kneaded product, water (F) may be added to the kneaded product of the respective components (A), (C), and (D) as needed. Thus, the progress of a reaction between the silane coupling agent (D) and the silica particles (C) can be more reliably made.

In addition, it is preferable to knead the respective components (A), (C), and (D)) by conducting a first step of heating the components to a first temperature and a second step of heating the components to a second temperature. Thus, in the first step, the silica particles (C) can be surface-treated with the coupling agent (D), and in the second step, by-products that are produced in the reaction between the silica particles (C) and the coupling agent (D) can be reliably removed from the kneaded product. Then, the component (A) may be added to the obtained kneaded product and further kneaded as needed. Thus, the fitting among the components of the kneaded product can be improved.

The first temperature is preferably, for example, about 40°C to 120°C, and more preferably, for example, about 60°C to 90°C. The second temperature is preferably, for example, about 130°C to 210°C, and more preferably, for example, about 160°C to 180°C.

In addition, the atmosphere in the first step is preferably an inert atmosphere such as a nitrogen atmosphere, and the atmosphere in the second step is preferably an atmosphere under reduced pressure.

Further, the time in the first step is preferably, for example, about 0.3 to 1.5 hours, and more preferably about 0.5 to 1.2 hours. The time in the second step is, for example, preferably about 0.7 to 3.0 hours, and more preferably about 1.0 to 2.0 hours.

By conducting the first step and the second step under the same conditions as above, the effect can be obtained more remarkably.

[2] Next, predetermined amounts of the organohydrogen polysiloxane (B) and the platinum or platinum compound (E) are weighed, and the kneaded product prepared in the step [1] is kneaded with the respective components (B) and (E) using an optional kneading device, thereby obtaining a silicone rubber-based curable composition. The obtained silicone rubber-based curable composition may be a paste including a solvent.

Furthermore, in a case where the respective components (B) and (E) are kneaded, it is preferable to knead the organohydrogen polysiloxane (B) with the kneaded product prepared in the step [1] and knead the platinum or platinum compound (E) with the kneaded product prepared in the step [1] in advance; and then knead the respective kneaded products. Thus, the respective components (A) to (E) can be reliably dispersed in the silicone rubber-based curable composition without the progress of the reaction between the vinyl group-containing organopolysiloxane (A) and the organohydrogen polysiloxane (B).

The temperature at which the respective components (B) and (E) are kneaded, which is a roll setting temperature, is preferably about 10°C to 70°C, and more preferably about 25°C to 30°C.

In addition, the kneading time is preferably about 5 minutes to 1 hour, and more preferably about 10 to 40 minutes.

By setting the temperature within the range in the steps [1] and [2], the progress of the reaction between the vinyl group-containing organopolysiloxane (A) and the organohydrogen polysiloxane (B) can be more reliably prevented or suppressed. In addition, by setting the kneading time within the range in the steps [1] and [2], the respective components (A) to (E) can be more reliably dispersed in the silicone rubber-based curable composition.

Furthermore, the kneading device used in each of the steps [1] and [2] is not particularly limited but, for example, a kneader, two rolls, a Banbury mixer (continuous kneader), a pressurization kneader, or the like can be used.

In addition, a reaction inhibitor such as 1-ethynylcyclohexanol may be added to the kneaded product in the present step [2]. Thus, even in a case where the temperature of the kneaded product is set to a relatively high temperature, the progress of the reaction between the vinyl group-containing organopolysiloxane (A) and the organohydrogen polysiloxane (B) can be more reliably prevented or suppressed.

[3] Next, silicone rubber is formed by curing the silicone rubber-based curable composition.

In the present embodiment, in a step of curing the silicone rubber-based curable resin composition, for example, after heating (primary curing) at 100°C to 250°C for 1 to 30 minutes, post baking (secondary curing) at 200°C for 1 to 4 hours is performed.

Silicone rubber consisting of a cured product of the silicone rubber-based curable resin composition is obtained by performing the above-described steps.

Further, [3] next, an insulating paste can be obtained by dissolving the silicone rubber-based curable composition obtained in the step [2] in a solvent.

In addition, [3] next, a conductive paste can be obtained by dissolving the silicone rubber-based curable composition obtained in the step [2] in a solvent and adding a conductive filler thereto.

### (Solvent)

The conductive paste and the insulating paste contains a solvent.

Various known solvents can be used as the solvent, and examples thereof include a high-boiling point solvent. These may be used alone or in combination of two or more kinds thereof.

The lower limit of the boiling point of the high-boiling point solvent is, for example, higher than or equal to 100°C, preferably higher than or equal to 130°C, and more preferably higher than or equal to 150°C. In such a case, it is possible to improve the printing stability of screen printing or the like. Meanwhile, the upper limit of the boiling point of the high-boiling point solvent is not particularly limited and may be, for example, equal to or lower than 300°C, equal to or lower than 290°C, or equal to or lower than 280°C. In this manner, an excessive thermal history during formation of a wiring can be suppressed, and thus damage to a base substrate or the shape of a wiring formed of the conductive paste can be satisfactorily maintained.

In addition, the solvent can be appropriately selected from the viewpoints of the solubility and the boiling point of the silicone rubber-based curable resin composition, and can include, for example, an aliphatic hydrocarbon having greater than or equal to 5 and less than or equal to 20 carbon atoms, preferably an aliphatic hydrocarbon having greater than or equal to 8 and less than or equal to 18 carbon atoms, and more preferably an aliphatic hydrocarbon having greater than or equal to 10 and less than or equal to 15 carbon atoms.

In addition, examples of the solvent include aliphatic hydrocarbons such as pentane, hexane, cyclohexane, heptane, methylcyclohexane, ethylcyclohexane, octane, decane, dodecane, and tetradecane; aromatic hydrocarbons such as benzene, toluene, ethylbenzene, xylene, mesitylene, trifluoromethylbenzene, and benzotrifluoride; ethers such as diethyl ether, diisopropyl ether, dibutyl ether, cyclopentyl methyl ether, cyclopentyl ethyl ether, ethylene glycol dimethyl ether, ethylene glycol diethyl ether, diethylene glycol dimethyl ether, diethylene glycol monobutyl ether, dipropylene glycol dimethyl ether, dipropylene glycol methyl-n-propyl ether, 1,4-dioxane, 1,3-dioxane, and tetrahydrofuran; haloalkanes such as dichloromethane, chloroform, 1,1-dichloroethane, 1,2-dichloroethane, 1,1,1-trichloroethane, and 1,1,2-trichloroethane; carboxylic acid amides such as N,N-dimethylformamide and N,N-dimethylacetamide; sulfoxides such as dimethyl sulfoxide and diethyl sulfoxide; and esters such as diethyl carbonate. These may be used alone or in combination of two or more kinds thereof.

The solvent used here may be appropriately selected from solvents capable of uniformly dissolving or dispersing the composition components in the conductive paste.

The solvent may include a first solvent in which the upper limit of the polarity term (δₚ) of the Hansen solubility parameter is, for example, less than or equal to 10 MPa^{1/2}, preferably less than or equal to 7 MPa^{1/2}, and more preferably less than or equal to 5.5 MPa^{1/2}. In this manner, the dispersibility and the solubility of the silicone rubber-based curable resin composition in the paste can be enhanced. The lower limit of the polarity term (δₚ) of the first solvent is not particularly limited, and may be, for example, greater than or equal to 0 Pa^{1/2}.

The upper limit of the hydrogen bond term (δₕ) of the Hansen solubility parameter in the first solvent is, for example, less than or equal to 20 MPa^{1/2}, preferably less than or equal to 10 MPa^{1/2}, and more preferably less than or equal to 7 MPa^{1/2}. In this manner, the dispersibility and the solubility of the silicone rubber-based curable resin composition in the paste can be enhanced. The lower limit of the hydrogen bond term (δₕ) of the first solvent is not particularly limited and may be, for example, greater than or equal to 0 Pa^{1/2}.

The Hansen solubility parameter (HSP) is an index that represents solubility, which indicates how much a certain substance dissolves in another substance. HSP represents solubility as a three-dimensional vector. This three-dimensional vector can be represented by a dispersion term (δ_{d}), a polarity term (δₚ), and a hydrogen bond term (δₕ). In addition, substances having similar vectors can be determined to be highly soluble in each other. The similarity between vectors can be determined by the distance of the Hansen solubility parameter (HSP distance).

The Hansen solubility parameter (HSP value) used in the present specification can be computed using software called HSPiP (Hansen Solubility Parameters in Practice). Here, the computer software HSPiP developed by Hansen and Abbott includes a function of computing the HSP distance and a database of the Hansen parameters of a variety of resins, solvents, and non-solvents.

The solubility of each resin in a pure solvent and a solvent mixture of a good solvent and a poor solvent is investigated, the results are input to the HSPiP software, and D: dispersion term, P: polarity term, H: hydrogen bond term, and R0: solubility sphere radius are computed.

As the solvent of the present embodiment, for example, a solvent having a small difference in the HSP distance and the polarity term or the hydrogen bond term from the elastomer or the constitution unit that constitutes the elastomer can be selected.

The lower limit of the viscosity of the conductive paste and/or the insulating paste in a case of measurement under conditions of a shear rate of 20 [1/s] at a room temperature of 25°C is, for example, greater than or equal to 1 Pa·s, preferably greater than or equal to 5 Pa·s, and more preferably greater than or equal to 10 Pa·s. In this manner, the film-forming property can be improved. In addition, the shape-retaining property during the formation of a thick film can be enhanced. Meanwhile, the upper limit of the viscosity of the conductive paste and/or the insulating paste at a room temperature of 25°C is, for example, less than or equal to 100 Pa·s, preferably less than or equal to 90 Pa·s, and more preferably less than or equal to 80 Pa·s. In this manner, the printability in the paste can be improved.

The viscosity measured under conditions of a shear rate of 1 [1/s] at a room temperature of 25°C is defined as η1, the viscosity measured under conditions of a shear rate of 5 [1/s] at a room temperature of 25°C is defined as η5, and a thixotropic index is defined as a viscosity ratio (η1/η5).

Here, the lower limit of the thixotropic index of the conductive paste and/or the insulating paste is, for example, greater than or equal to 1.0, preferably greater than or equal to 1.1, and more preferably greater than or equal to 1.2. In this manner, the shape of the wiring obtained by a printing method can be stably maintained. Meanwhile, the upper limit of the thixotropic index of the conductive paste and/or the insulating paste is, for example, less than or equal to 3.0, preferably less than or equal to 2.5, and more preferably less than or equal to 2.0. In this manner, the printability of the paste can be improved.

The content of the silicone rubber-based curable composition in the insulating paste is preferably greater than or equal to 10% by mass, more preferably greater than or equal to 15% by mass, and still more preferably greater than or equal to 20% by mass with respect to 100% by mass of the insulating paste. In addition, the content of the silicone rubber-based curable composition in the insulating paste is preferably less than or equal to 50% by mass, more preferably less than or equal to 40% by mass, and still more preferably less than or equal to 35% by mass with respect to 100% by mass of the insulating paste.

### (Conductive filler)

As the conductive filler, a known conductive material may be used, or a metal powder (G) may be used.

The metal that constitutes the metal powder (G) is not particularly limited, and the metal powder may contain at least one or two or more kind of copper, silver, gold, nickel, tin, lead, zinc, bismuth, antimony, and metal powder obtained by alloying these metals.

Among these, it is preferable that the metal powder (G) contains silver or copper, that is, silver powder or copper powder from the viewpoints of high conductivity and high availability.

Further, the metal powder (G) coated with a different kind of metal can also be used.

In the present embodiment, the shape of the metal powder (G) is not limited, and metal powder having a dendrite shape, a spherical shape, a phosphorus piece shape, or the like that has been used in the related art can be used. Among these, a scale-like metal powder (G) may also be used.

In addition, the particle diameter of the metal powder (G) is also not limited, but the average particle diameter D₅₀ thereof is, for example, preferably greater than or equal to 0.001 pm, more preferably greater than or equal to 0.01 um, and still more preferably greater than or equal to 0.1 µm. In regard to the particle diameter of the metal powder (G), the average particle diameter D₅₀ thereof is, for example, preferably less than or equal to 1,000 um, more preferably less than or equal to 100 pm, and still more preferably less than or equal to 20 um.

In a case where the average particle diameter D₅₀ thereof is set to be in the above-described ranges, the silicone rubber can exhibit appropriate conductivity.

Further, the particle diameter of the metal powder (G) can be defined, for example, as an average value of 200 optionally selected metal powders by performing image analysis after observing a conductive paste or silicone rubber molded using the conductive paste with a transmission electron microscope or the like.

The content of the conductive filler in the conductive paste is preferably greater than or equal to 20% by mass, more preferably greater than or equal to 30% by mass, and still more preferably greater than or equal to 40% by mass with respect to the entire conductive paste. In addition, the content of the conductive filler in the conductive paste is preferably less than or equal to 85% by mass, more preferably less than or equal to 75% by mass, and still more preferably less than or equal to 65% by mass with respect to the entire conductive paste.

In a case where the content of the conductive filler is set to be greater than or equal to the above-described lower limits, the silicone rubber can have appropriate conduction characteristics. Further, in a case where the content of the conductive filler is set to be less than or equal to the above-described upper limits, the silicone rubber can have appropriate flexibility.

The content of the silicone rubber-based curable composition in the conductive paste is preferably greater than or equal to 1% by mass, more preferably greater than or equal to 3% by mass, and still more preferably greater than or equal to 5% by mass with respect to 100% by mass of the conductive paste. In addition, the content of the silicone rubber-based curable composition in the conductive paste is preferably less than or equal to 25% by mass, more preferably less than or equal to 20% by mass, and still more preferably less than or equal to 15% by mass with respect to 100% by mass of the conductive paste.

In a case where the content of the silicone rubber-based curable composition is set to be greater than or equal to the above-described lower limits, the silicone rubber can have appropriate flexibility. Further, in a case where the content of the silicone rubber-based curable composition is set to be less than or equal to the above-described upper limits, the mechanical strength of the silicone rubber can be improved.

The lower limit of the content of the silica particles (C) in the conductive paste may be, for example, greater than or equal to 1% by mass, preferably greater than or equal to 3% by mass, and more preferably greater than or equal to 4% by mass with respect to 100% by mass of the total amount of the silica particles (C) and the conductive filler. In this manner, the mechanical strength of the silicone rubber can be improved. Meanwhile, the upper limit of the content of the silica particles (C) in the conductive paste is, for example, less than or equal to 20% by mass, preferably less than or equal to 15% by mass, and more preferably less than or equal to 10% by mass with respect to 100% by mass of the total amount of the silica particles (C) and the conductive filler. In this manner, the balance between the elastic electrical characteristics and the mechanical strength of the silicone rubber can be achieved.

The lower limit of the content of the conductive filler in the conductive cured product obtained by curing the conductive paste constituting the conductive elastomer layer 20 is, for example, greater than or equal to 50% by mass, preferably greater than or equal to 60% by mass, and more preferably greater than or equal to 70% by mass with respect to 100% by mass of the conductive cured product. In this manner, the elastic electrical characteristics can be improved. Meanwhile, the upper limit of the content of the conductive filler in the conductive cured product is, for example, less than or equal to 90% by mass, preferably less than or equal to 88% by mass, and more preferably less than or equal to 85% by mass with respect to 100% by mass of the conductive elastomer layer 20. In this manner, degradation of rubber properties such as elasticity can be suppressed.

The lower limit of the content of the silica particles (C) in the conductive cured product obtained by curing the conductive paste constituting the conductive elastomer layer 20 may be, for example, greater than or equal to 1% by mass, preferably greater than or equal to 3% by mass, and more preferably greater than or equal to 4% by mass with respect to 100% by mass of the total amount of the silica particles (C) and the conductive filler. In this manner, the mechanical strength of the silicone rubber can be improved. Meanwhile, the upper limit of the content of the silica particles (C) in the conductive cured product is, for example, less than or equal to 20% by mass, preferably less than or equal to 15% by mass, and more preferably less than or equal to 10% by mass with respect to 100% by mass of the total amount of the silica particles (C) and the conductive filler. In this manner, the balance between the elastic electrical characteristics and the mechanical strength of the silicone rubber can be achieved.

The embodiments of the present invention have been described above, but these are examples of the present invention and various configurations other than the above can be adopted. In addition, the present invention is not limited to the above-described embodiments, and modifications, improvements, and the like can be made within a range where the object of the present invention can be achieved.

### EXAMPLES

Hereinafter, examples of the present invention will be described in detail. However, the present invention is not limited to the description of the examples.

The raw material components listed in Table 1 are shown below.
(A1-1): first vinyl group-containing linear organopolysiloxane: vinyl group-containing dimethylpolysiloxane synthesized by synthesis scheme 1 shown below (structure represented by Formula (1-1))
(A1-2): second vinyl group-containing linear organopolysiloxane: vinyl group-containing dimethylpolysiloxane synthesized by synthesis scheme 2 shown below (structure in which R¹ and R² in the structure represented by Formula (1-1) represent a vinyl group)

### (Organohydrogen polysiloxane (B))

(B-1): organohydrogen polysiloxane: "TC-25D", manufactured by Momentive Performance Materials Inc.

### (Silica particles (C))

(C): silica fine particles (particle diameter: 7 nm, specific surface area: 300 m²/g), "AEROSIL 300", manufactured by Nippon Aerosil Co., Ltd.

### (Silane coupling agent (D))

(D-1): hexamethyldisilazane (HMDZ), "HEXAMETHYLDISILAZAE" (SIH6110.1)", manufactured by Gelest, Inc.
(D-2): divinyltetramethyldisilazane, "1,3-DIVINYLTETRAMETHYLDISILAZANE (SID4612.0)", manufactured by Gelest, Inc.

### (Platinum or Platinum Compound (E))

(E-1): platinum compound (trade name "TC-25A", manufactured by Momentive Performance Materials Inc.)

### (Water (F))

(F): pure water

### (Metal Powder (G))

(G1): silver powder, trade name "TC-101", manufactured by Tokuriki Honten Co., Ltd, median size d₅₀: 8.0 um, aspect ratio: 16.4, average long diameter: 4.6 µm

### (Synthesis of vinyl group-containing organopolysiloxane (A))

### [Synthesis scheme 1: synthesis of first vinyl group-containing linear organopolysiloxane (A1-1)]

The first vinyl group-containing linear organopolysiloxane (A1-1) was synthesized according to the following Formula (5).

That is, 74.7 g (252 mmol) of octamethylcyclotetrasiloxane and 0.1 g of potassium siliconate were added to 300 mL separable flask including a cooling tube and a mixing impeller where the atmosphere was replaced with Ar gas. The solution was heated and stirred at 120°C for 30 minutes. At this time, an increase in viscosity was observed.

Next, the solution was heated up to 155°C and was continuously stirred for 3 hours. After 3 hours, 0.1 g (0.6 mmol) of 1,3-divinyltetramethyldisiloxane was added and was further stirred at 155°C for 4 hours.

Further, after 4 hours, the solution was diluted with 250 mL of toluene, and was cleaned with water 3 times. After cleaning, the organic layer was cleaned with 1.5 L of methanol and was purified by precipitation to separate an oligomer and a polymer. The obtained polymer was dried at 60°C under reduced pressure overnight, thereby obtaining a first vinyl group-containing linear organopolysiloxane (A1-1) (Mn = 2.2 × 10⁵, Mw = 4.8 × 10⁵). In addition, the vinyl group content calculated by H-NMR spectrum measurement was 0.04% by mole.

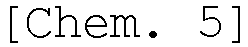

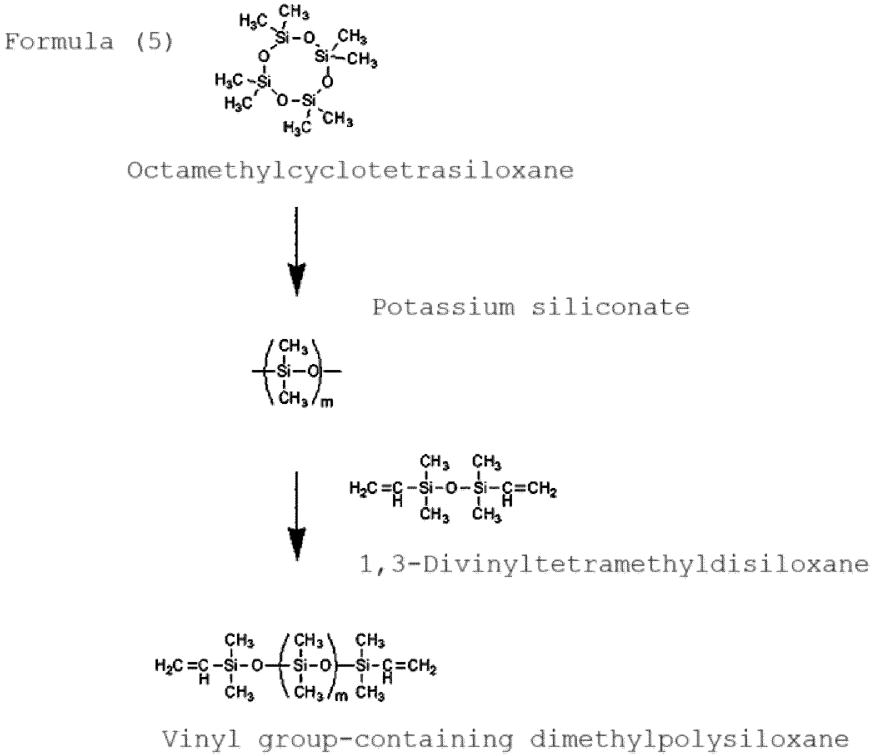

### [Synthesis scheme 2: synthesis of second vinyl group-containing linear organopolysiloxane (A1-2)]

A second vinyl group-containing linear organopolysiloxane (A1-2) was synthesized (Mn = 2.3 × 10⁵, Mw = 5.0 × 10⁵) as shown in Formula (6) in the same manner as in the synthesis step for (A1-1) except that 0.86 g (2.5 mmol) of 2,4,6,8-tetramethyl 2,4,6,8-tetravinylcyclotetrasiloxane was used in addition to 74.7 g (252 mmol) of octamethylcyclotetrasiloxane in the synthesis step for (A1-1). Further, the content of the vinyl group calculated by H-NMR spectrum measurement was 0.93% by mole.

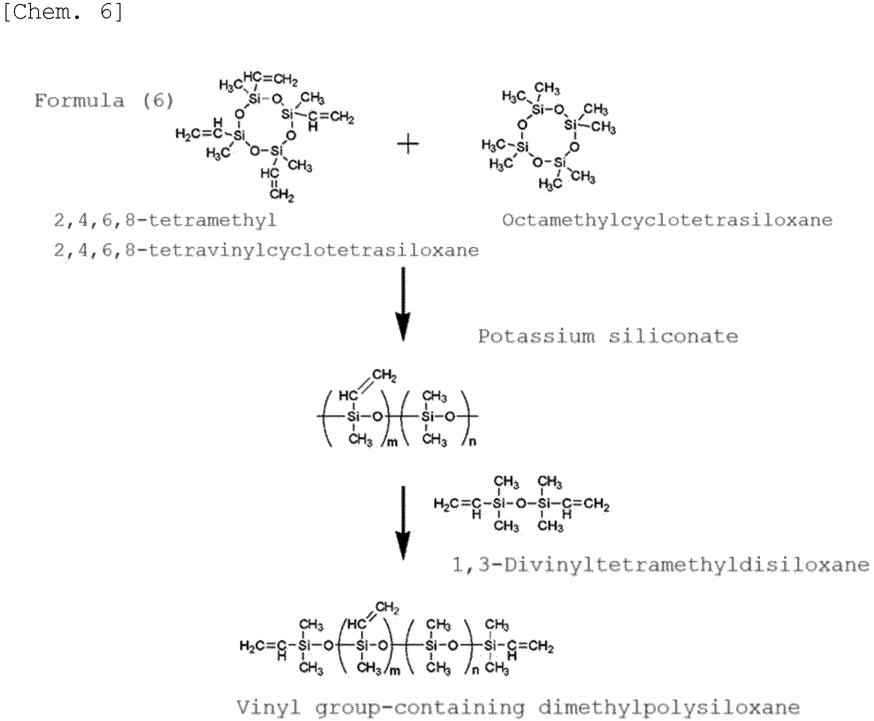

### (Preparation of silicone rubber-based curable composition)

The silicone rubber-based curable compositions of samples 1 and 2 were adjusted according to the following procedure.

First, a mixture including 90% of the vinyl group-containing organopolysiloxane (A), the silane coupling agent (D), and the water (F) at a ratio shown in Table 1 below was kneaded in advance. Next, the silica particles (C) were further added to the mixture and kneaded. As a result, a kneaded product (silicone rubber compound) was obtained.

Here, the kneading after the addition of the silica particles (C) was performed through a first step of kneading the components under conditions of 60°C to 90°C in a nitrogen atmosphere for the coupling reaction and a second step of kneading the components under conditions of 160°C to 180°C for 2 hours in a reduced pressure atmosphere for removing the by-product (ammonia). Next, the kneaded product was cooled, the remaining 10% of the vinyl group-containing organopolysiloxane (A) was dividedly added twice, and the components were kneaded for 20 minutes.

Subsequently, the organohydrogen polysiloxane (B) and the platinum or the platinum compound (E) were added to 100 parts by weight of the obtained kneaded product (silicone rubber compound) with the proportions listed in Table 2, and the mixture was kneaded with a roll, thereby obtaining a silicone rubber-based curable composition.

### (Preparation of conductive paste)

15.3 parts by weight of the silicone rubber-based curable composition of the obtained sample 1 was immersed in 34.8 parts by weight of decane (solvent), and stirred with a rotating and revolving mixer, 65.2 parts by weight of metal powder (G1) was added thereto, and the mixture was further kneaded with a rotating and revolving mixer, thereby obtaining a conductive paste in which the total content of the silicone rubber-based curable composition and the metal powder (G1) was 69.8% by weight.

### (Preparation of insulating paste)

15.3 parts by weight of the silicone rubber-based curable composition of the obtained sample 2 was immersed in 32.5 parts by weight of decane (solvent), and stirred with a rotating and revolving mixer, thereby obtaining an insulating paste.

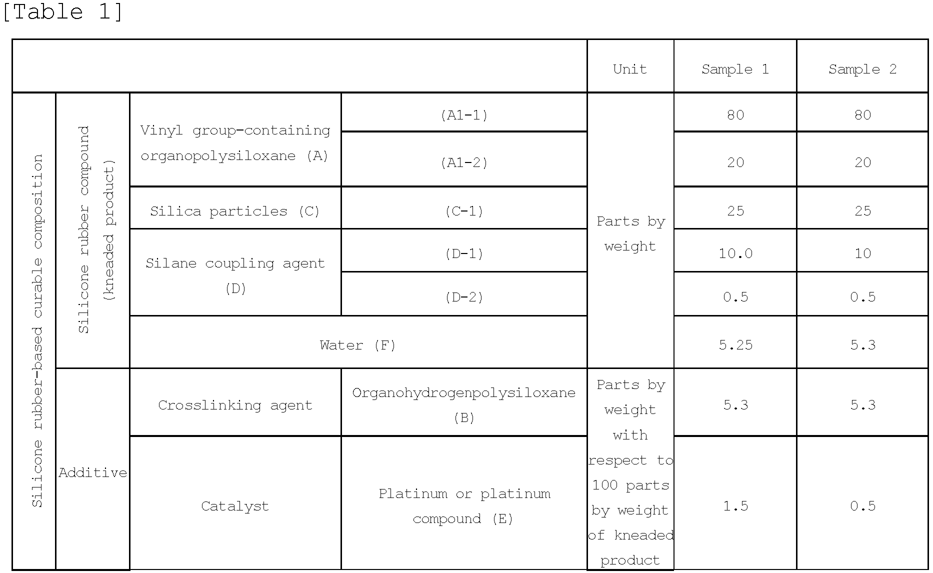

### (Preparation of flexible sheet electrode)

A flexible base material (knitted fabric made of polyester, basis weight of 130 g/m²) having a size of 7 cm × 7 cm × 0.4 mmt was placed on a plane of a SUS substrate, and a mask (made of polyethylene terephthalate) having a thickness of 125 um and one opening with a size of 5 cm × 5 cm was placed on a surface of the flexible base material.

Subsequently, the opening of the mask was coated with the above-described insulating paste, squeegee printing was performed using a glass plate and the insulating paste based on the printing conditions listed in Table 2 in a printing direction for vertical printing orthogonal to the mesh of the fibers, the mask was removed, and the resultant was dried at 140°C for 20 minutes. Subsequently, squeegee printing was performed using a conductive paste. Here, in Examples 1 and 2 and Comparative Examples 1 and 2, the squeegee printing was performed using only the conductive paste.

Subsequently, the mask was removed, and the printed insulating paste and conductive paste were dried at 140°C for 20 minutes and cured at 180°C for 2 hours, thereby preparing a flexible sheet electrode including an elastomer electrode layer provided over the flexible base material.

In Table 2, the adhesion amount of the insulating elastomer layer which was a cured product of the insulating paste and the adhesion amount of the conductive elastomer layer which was a cured product of the conductive paste were calculated by subtracting the weight of the flexible base material, which had been measured in advance, from the weight of the flexible sheet electrode.

In addition, the flexible sheet electrode was cut in the lamination direction, one cross section thereof was observed with an electron microscope to acquire an SEM image with a 1 mm × 1 mm region, and the thickness (T1) of an impregnation layer in which the inside of the flexible base material was impregnated with the elastomer electrode layer, the thickness (T2) of the lamination layer in which the inside of the flexible base material was not impregnated with the elastomer electrode layer, and the thickness (T3) of the flexible base material were determined based on the SEM image.

Specifically, a cross-sectional SEM image A of only the flexible base material in a region where the conductive elastomer layer was not formed was acquired, and the thickness (T3) of the base material was measured from the SEM image A.

A cross-sectional SEM image B of the flexible sheet electrode in the region where the conductive elastomer layer was formed was acquired, the total thickness (X) of the flexible sheet electrode was measured from the SEM image B, and the thickness (T2) of the lamination layer was calculated by subtracting the thickness (T3) of the base material from the total thickness (X).

The length from a boundary where the layer was impregnated with the insulating paste or the conductive paste to the boundary between the base material and the lamination layer on the cross-sectional SEM image B of the flexible sheet electrode was measured as the thickness (T1) of the impregnation layer.

In addition, an SEM image with a 1 mm × 1 mm region on the surface of the conductive elastomer layer was confirmed.

As a result, in Examples 1 to 4, it was found that the conductive elastomer layer in the above-described region was formed by covering the entire surface of the flexible base material, and the surface thereof was formed of a smooth surface smoother than the surface structure of the flexible base material.

On the contrary, in Comparative Example 1, it was found that the conductive elastomer layer in the above-described region had a portion that adhered to follow the surface structure of the flexible base material of the base substrate, and one surface was not formed as a whole.

**[Table 2]**

| Flexible sheet electrode | | | Unit | Comparative Example 1 | Example 1 | Example 2 |
|---|---|---|---|---|---|---|
| Printing conditions | Insulating paste | Number of lamination layers of mask | | 0 | 0 | 0 |
| | | Number of times of printing | | 0 | 0 | 0 |
| | | Adhesion amount | mg/cm² | 0 | 0 | 0 |
| | Conductive paste | Number of lamination layers of mask | | 1 | 4 | 6 |
| | | Number of times of p rinting | | 1 | 1 | 1 |
| | | Adhesion amount | mg/cm² | 24.5 | 73.3 | 104.8 |
| Thickness T3 of flexible base material | | | µm | 408 | 408 | 408 |
| Elastomer electrode layer | Thickness T1 of impregnation layer | | µm | 155 | 167 | 191 |
| | Thickness T2 of lamination layer | | µm | 0 | 103 | 199 |
| T1/T2 | | | | - | 1.6 | 1.0 |
| T1/T3 | | | | 0.38 | 0.41 | 0.47 |
| Surface resistivity of conductive elastomer layer | Initial surface resistivity of conductive elastomer layer | | Ω/□ | 5.3E-02 | 7.8E-03 | 4.2E-03 |
| | Surface resistivity of conductive elastomer layer after washing test | | Ω/□ | 0.47 | 1.7E-02 | 7.6E-03 |
| Tensile test | 250% tensile test force (at 250% elongation) | | N | 23.6 | 28.8 | 27.5 |
| | 20% tensile test force (at 20% elongati on) | | N | 1.4 | 2.8 | 3.8 |
| | 100% tensile test force (at 100% elonga tion) | | N | 4.6 | 6.4 | 7.8 |
| Ease of follow-up deformation | | | | Satisfactory | Satisfactory | Satisfactory |

**[Continued Table 2]**

| Flexible sheet electrode | | | Unit | Example 3 | Example 4 | Comparative Example 2 |
|---|---|---|---|---|---|---|
| Printing conditions | Insulating paste | Number of lamination layers of mask | | 6 | 6 | 0 |
| | | Number of times of printing | | 1 | 1 | 0 |
| | | Adhesion amount | mg/cm² | 24.9 | 24.9 | 0 |
| | Conductive paste | Number of laminati on layers of mask | | 2 | 4 | 10 |
| | | Number of times of printing | | 1 | 1 | 1 |
| | | Adhesion amount | mg/cm² | 23.5 | 47.0 | - |
| Thickness T3 of flexible base material | | | µm | 408 | 408 | 408 |
| Elastomer electrode layer | Thickness T1 of impregnation layer | | µm | 408 | 408 | - |
| | Thickness T2 of lamination layer | | µm | 190 | 290 | 280 |
| T1/T2 | | | | 2.1 | 1.4 | - |
| T1/T3 | | | | 1 | 1 | - |
| Surface resistivity of conductive elastomer layer | Initial surface resistivity of conductive elastomer layer | | Ω/□ | 1.7E-01 | 1.5E-02 | - |
| | Surface resistivity of conductive elastomer layer after washing test | | Ω/□ | 1.3E-01 | 9.8E-03 | - |
| Tensile test | 250% tensile test force (at 250% elongation) | | N | 26.9 | 26.5 | 32.0 |
| | 20% tensile test force (at 20 % elongation) | | N | 2.2 | 3.5 | 5.8 |
| | 100% tensile test force (at 1 00% elongation) | | N | 5.6 | 7.3 | 10.4 |
| Ease of follow-up deformation | | | | Satisfactory | Satisfactory | Unsatisfactory |

The following items of the obtained flexible sheet electrode were evaluated. The results are listed in Table 2.

### (Tensile test)

The obtained flexible sheet electrode was cut out to prepare a sample test piece 3 having a rectangular shape shown in Fig. 6.

The dimensions of the printed portion (conductive elastomer layer 20 provided on the surface side of the elastomer electrode layer 2) of the sample test piece 3 were 10 mm × 20 mm, and the dimensions of the flexible base material 10 were 15 mm × 50 mm.

The printed portion of the sample test piece 3 was held by chucks of a tensile testing machine in a state of being tightly stretched such that the center of the printed portion was positioned at the center between the chucks, and a tensile test was performed under the conditions of a distance of 10 mm between the chucks, a stroke (elongation ratio) of 250% (elongation from the initial 10 mm to 35 mm), and a tensile rate of 250 mm/min.

A tensile test force (N) at 20% elongation, at 100% elongation, and at 250% elongation was measured. The results are listed in Table 2.

### (Surface resistivity)

The obtained printed portion of the flexible sheet electrode was cut out into a size of 1 cm × 1 cm to prepare a sample.

The surface resistivity (Ω/□) of the conductive elastomer layer on the surface side of the elastomer electrode layer was measured using the sample by placing the probe to the central portion in a state of non-elongation in an environment of 25°C using a four-probe method.

### (Washing resistance)

The obtained flexible sheet electrode was subjected to the following washing test.
1. The flexible sheet electrode was stretched and contracted by 50% 300 times in each of the vertical and horizontal directions.
2. A neutral detergent and water were put into a beaker, and the flexible sheet electrode which had been subjected to the treatment of 1 was immersed therein, followed by stirring for 24 hours.
3. Water was put into a beaker, the flexible sheet electrode subjected to the treatment of 2 was immersed therein, and the mixture was stirred for 10 minutes and rinsed with a detergent.
4. The resultant was dried in an oven at 90°C for 30 minutes.

The above-described procedures 1 to 4 were repeated 50 times.

The surface resistivity of the conductive elastomer layer was measured for the flexible sheet electrodes of each examples and each comparative example after the above-described washing test based on the above-described measurement conditions.

In addition, in each example, it was confirmed that the initial state was maintained without occurrence of cracks in the conductive elastomer layer or occurrence of peeling between the conductive elastomer layer and the flexible base material. On the contrary, in Comparative Example 1, cracks in the conductive elastomer layer and peeling between the conductive elastomer layer and the flexible base material were confirmed.

### (Ease of follow-up deformation)

The flexible base material of the flexible sheet electrode obtained in each example and each comparative example described above was sewn to a chest portion of a T-shirt with a sewing thread. The subject put on and took off the T-shirt to which the flexible sheet electrode was sewn a plurality of times.

In Examples 1 to 4 and Comparative Example 1, it was confirmed that the flexible sheet electrode followed the chest of the subject during the mounting, and the wearable bioelectrode was not peeled off from the T-shirt after the putting on and taking off. In addition, the wearing feeling of the plurality of subjects was usually "there is a contact feeling, but it is not noticeable" and "there is no pain, but there is a discomfort", and it was confirmed that the feeling of wearing discomfort was relatively small. Therefore, the ease of follow-up deformation was evaluated as satisfactory.

On the contrary, in Comparative Example 2, a case where the wearable bioelectrode was peeled off from the T-shirt after the putting on and taking off was found, and the wearing feeling of a plurality of the subjects was "feeling pain or feeling a significant discomfort", and it was confirmed that the feeling of wearing discomfort was relatively large. Therefore, the ease of follow-up deformation was evaluated as poor.

### (Measurement of biological potential)

An external connection portion (a male snap button made of a metal) was mounted on the other surface of the flexible base material on a side opposite to the surface on which the conductive elastomer layer was formed in the obtained flexible sheet electrode of each example, and the external connection portion and the conductive elastomer layer were electrically connected to each other, thereby obtaining a wearable bioelectrode.

Three wearable bioelectrodes were prepared, and each of the external connection portions of the three wearable bioelectrodes was connected to BITalino (manufactured by Plux) via an electrode cable with a connector (female snap button) and an ECG sensor (manufactured by Plux), thereby preparing an electrocardiogram measuring device (biosensor).

The flexible sheet electrode provided on one surface of the three wearable bioelectrodes was directly mounted on the skin of the subject, and the cardiac potential of the subject was measured by a three-point induction method (the measurement point, the reference, and the body earth).

As shown in the above-described results, it was confirmed that the electrocardiogram waveform could be monitored by using the wearable bioelectrode including the flexible sheet electrode of each example.

The flexible sheet electrodes of Examples 1 to 4 had excellent conduction characteristics after washing as compared with Comparative Example 1 and exhibited excellent ease of follow-up deformation as compared with Comparative Example 2. The flexible sheet electrode of each example can be suitably used for a wearable bioelectrode.

This application claims priority on the basis of Japanese Patent Application No. 2022-010456 filed on January 26, 2022, the entire disclosure of which is incorporated into the present specification by reference.

### REFERENCE SIGNS LIST

- 1: flexible sheet electrode
- 2: elastomer electrode layer
- 3: sample test piece
- 10: flexible base material
- 11: one surface
- 12: main body
- 13: other surface
- 14: extension portion
- 20: conductive elastomer layer
- 20a: conductive elastomer layer
- 20b: conductive elastomer layer
- 21: impregnation layer
- 22: one surface
- 23: lamination layer
- 24: elastic wiring
- 26: connection portion
- 30: external connection portion
- 32: attachment plate
- 34: attachment pin
- 50: protective layer
- 52: insulating protective layer
- 60: insulating elastomer layer
- 60a: insulating elastomer layer
- 60b: insulating elastomer layer
- 100: wearable bioelectrode
- 200: biosensor
- 210: connector
- 220: cable
- 230: sensor module
- 240: computer

## Claims

1. A flexible sheet electrode comprising:
a flexible base material; and
an elastomer electrode layer provided over the flexible base material,
wherein the elastomer electrode layer is formed over an outside of the flexible base material and includes a lamination layer including a conductive elastomer layer, and
in a case where a tensile test is performed on the flexible sheet electrode according to the following procedure, a 100% tensile test force is greater than or equal to 5.0 N and less than or equal to 10.0 N,
(procedure)
a sample test piece having a rectangular shape is prepared from the flexible sheet electrode,
in a case where the elastomer electrode layer of the sample test piece is held by chucks of a tensile testing machine such that a center of the elastomer electrode layer is positioned at a center between the chucks, and a distance between the chucks is elongated from 0% to 250% under a condition of a tensile rate of 250 mm/min, a tension (N) is measured during extension of the distance by X%, and a value of the tension is defined as the X% tensile test force.

2. The flexible sheet electrode according to Claim 1,
wherein in a case where the tensile test is performed on the flexible sheet electrode according to the procedure, a 20% tensile test force is greater than or equal to 1.5 N and less than or equal to 5.5 N.

3. The flexible sheet electrode according to Claim 1 or 2, further comprising:
an impregnation layer formed over an inside of the flexible base material,
wherein the impregnation layer includes the conductive elastomer layer and/or an insulating elastomer layer.

4. The flexible sheet electrode according to any one of Claims 1 to 3,
wherein the conductive elastomer layer contains a conductive filler and a non-conductive filler.

5. The flexible sheet electrode according to Claim 4,
wherein the conductive filler contains scale-like silver powder.

6. The flexible sheet electrode according to Claim 4 or 5,
wherein the non-conductive filler contains silica particles.

7. The flexible sheet electrode according to any one of Claims 4 to 6,
wherein a content of the conductive filler in the conductive elastomer layer is greater than or equal to 50% by mass and less than or equal to 90% by mass.

8. The flexible sheet electrode according to any one of Claims 1 to 7,
wherein the conductive elastomer layer contains silicone rubber.

9. The flexible sheet electrode according to any one of Claims 1 to 8,
wherein the flexible base material is woven fabric or knitted fabric.

10. A wearable bioelectrode comprising:
the flexible sheet electrode according to any one of Claims 1 to 9.

11. A biosensor comprising:
the wearable bioelectrode according to Claim 10.
